# EUROPEAN PATENT APPLICATION

(11) **EP 4 617 362 A1**
(43) Date of publication of application: **17.09.2025**
(21) Application number: 23888732.7
(22) Date of filing: 08.11.2023
(51) Int. Cl.: C12N 5/0735, C12N 5/10, C12N 15/09

(54) **METHOD FOR PRODUCING PLURIPOTENT STEM CELLS AND METHOD FOR INDUCING DIFFERENTIATION OF PLURIPOTENT STEM CELLS**

(30) Priority: 09.11.2022 JP 2022179472
(71) Applicant: Kaneka Corporation, Osaka-shi, Osaka 530-8288 (JP); RIKEN, Wako-shi, Saitama 351-0198 (JP)
(72) Inventor: KAMBAYASHI, Sho, Kobe-shi, Hyogo 650-0047 (JP); HAYASHI, Yohei, Tsukuba-shi, Ibaraki 305-0074 (JP); TAKASAKI, Mami, Tsukuba-shi, Ibaraki 305-0074 (JP)
(74) Representative: Hoffmann Eitle
(86) International application number: PCT/JP2023/040192
(87) International publication number: WO 2024/101385

(57) **Abstract**

A pluripotent stem cell that has higher efficiency of differentiation and is still inhibited from spontaneously differentiating into all three germ layers is produced. A pluripotent stem cell is suspension-cultured in a liquid medium containing a PKCβ inhibitor and a WNT inhibitor, and then suspension-cultured in a liquid medium not substantially containing a PKCβ inhibitor and/or a WNT inhibitor.

## Description

### Technical Field

The present invention relates to a method for producing a pluripotent stem cell by suspension culture and a method for inducing differentiation of the pluripotent stem cell produced by the production method.

### Background Art

Pluripotent stem cells such as ES cells and iPS cells have the ability to grow infinitely and the ability to differentiate into various somatic cells. The practical application of a therapy in which a somatic cell produced by inducing the differentiation of a pluripotent stem cell is transplanted has the potential to fundamentally reform a therapy for an incurable disease and a lifestyle-related disease. For example, technologies have already been developed for inducing differentiation of pluripotent stem cells into various somatic cells such as nerve cells, myocardial cells, blood cells, and retinal cells at an *in vitro* level.

On the other hand, regenerative medicine using pluripotent stem cells still has problems to be addressed for practical use, and one of the problems is the productivity of pluripotent stem cells per se. For example, it is said that approximately 2 × 10¹¹ cells are required for liver regeneration. Methods for culturing pluripotent stem cells are roughly classified into adherent culture in which cells are allowed to adhere to a flat culture substrate (plate) and cultured, and suspension culture in which cells are cultured by suspending the cells in a liquid medium. To culture the above-described number of cells by adherent culture, a culture substrate (plate) 10⁶ cm² or more is required. Such a substrate corresponds to approximately 20,000 ordinary 10 cm dishes. In this manner, the number of cells obtained by adherent culture on the surface of a culture substrate (plate) depends on the culture area, and thus, a vast area is required for scale-up, and it is difficult to supply cells in an amount necessary for regenerative medicine. On the other hand, in suspension culture, cells are cultured while suspended in a liquid medium, and thus, the number of cells to be obtained depends on the volume of the medium. Accordingly, it is considered that the scale-up of suspension culture is relatively realistic, and suitable for the mass production of cells. For example, Non-Patent Literature 1 discloses a suspension culture method in which, using a spinner flask cell as a cell culture container for suspension culture, pluripotent stem cells are cultured while a liquid medium is being stirred.

In addition, another problem facing the practical application of regenerative medicine is the productivity of a somatic cell of interest. To efficiently produce a somatic cell of interest, efforts have been made to improve the efficiency of induction of differentiation by culturing high-quality pluripotent stem cells, and various methods have been reported. For example, Non-Patent Literature 2 discloses a method for suppressing spontaneous differentiation of pluripotent stem cells by adding a protein kinase C (PKC) inhibitor to a medium in adherent culture of pluripotent stem cells. In addition, Non-Patent Literature 3 discloses a method for suppressing spontaneous differentiation of pluripotent stem cells by adding a tankyrase (TNKS) inhibitor to a medium in adherent culture of pluripotent stem cells.

Furthermore, Patent Literature 1 discloses a method in which, in suspension culture of a pluripotent stem cell, a PKCβ inhibitor and a TNKS inhibitor, which is a WNT inhibitor, are allowed to coexist, whereby spontaneous differentiation into all the three germ layers is suppressed, so that the undifferentiated state of the pluripotent stem cell is maintained.

### Citation List

### Non-Patent Literature

Non-Patent Literature 1: Olmer R. et al., Tissue Engineering: Part C, Volume 18 (10): 772-784 (2012)
Non-Patent Literature 2: M. Kinehara et al., PLoS One. 2013; 8(1): e54122
Non-Patent Literature 3: T. Sumi et al., PLoS One. 2013; 8(5): e63378

### Patent Literature

Patent Literature 1: WO2021/162090

### Summary of Invention

### Technical Problem

As described above, there has been developed a technology in which spontaneous differentiation of a pluripotent stem cell into all the three germ layers is suppressed by suspension-culturing the pluripotent stem cell in a medium containing a PKCβ inhibitor and a WNT inhibitor. However, in a case where a pluripotent stem cell obtained by applying this technology is differentiated into a cell of interest, there is a problem in that the efficiency of differentiation is decreased, compared with a pluripotent stem cell cultured by suspension culture in a medium not containing any of a PKCβ inhibitor and a WNT inhibitor.

In view of this, an object of the present invention is as follows: to produce a pluripotent stem cell with highly maintained efficiency of differentiation, simultaneously suppressing spontaneous differentiation of the cell into all the three germ layers; and to achieve induction of differentiation with excellent efficiency of differentiation by using the pluripotent stem cell obtained.

### Solution to Problem

The present inventors have made studies vigorously to achieve the above-described object, and have consequently come to complete the present invention through the discovery of a culture condition for improving the efficiency of differentiation in suspension culture during culture for inducing differentiation, in which a step of suspension-culturing a pluripotent stem cell in a medium containing a PKCβ inhibitor and a WNT inhibitor is maintained, in order to maintain the undifferentiated state of the cell.

The present invention encompasses the following:
(1) A method for producing a pluripotent stem cell, including:
   a step of suspension-culturing the pluripotent stem cell in a first liquid medium containing a PKCβ inhibitor and a WNT inhibitor; and
   a subsequent step of suspension-culturing the pluripotent stem cell in a second liquid medium not substantially containing a PKCβ inhibitor and/or a WNT inhibitor.
(2) The production method according to (1), wherein the second liquid medium does not substantially contain any one of the PKCβ inhibitor and the WNT inhibitor, and contains the other.
(3) The production method according to (1), including a step of changing the first liquid medium to the second liquid medium.
(4) The production method according to (1), wherein the concentration of the WNT inhibitor contained in the second liquid medium is less than 90 nM.
(5) The production method according to (1), wherein the concentration of the PKCβ inhibitor contained in the second liquid medium is less than 25 nM.
(6) The production method according to (1), wherein the first liquid medium contains the WNT inhibitor with the concentration of 90 nM or more and 40 µM or less and the PKCβ inhibitor with the concentration of 25 nM or more and 15 µM or less.
(7) The production method according to (1), wherein a cell population in which the proportion of cells positive for OCT4 is 90% or more, the proportion of cells positive for SOX2 is 90% or more, and the proportion of cells positive for NANOG is 90% or more is produced by the step of suspension-culturing the pluripotent stem cell in the second liquid medium.
(8) The production method according to (1), wherein the second liquid medium is a medium containing a PKCβ inhibitor and not substantially containing a WNT inhibitor, and wherein the pluripotent stem cell suitable for differentiation into a mesendodermal cell is produced by the step of suspension-culturing the pluripotent stem cell in the second liquid medium.
(9) The production method according to (1), wherein the second liquid medium is a medium containing a WNT inhibitor and not substantially containing a PKCβ inhibitor, and wherein the pluripotent stem cell suitable for differentiation into an ectodermal cell is produced by the step of suspension-culturing the pluripotent stem cell in the second liquid medium.
(10) A method for inducing differentiation of a pluripotent stem cell, including a step of culturing, in a medium for inducing differentiation, the pluripotent stem cell produced by the production method according to any one of (1) to (9).

The present specification encompasses the contents described in the specification and/or drawings of Japanese Patent Application No. 2022-179472, on which the priority of the present application is based.

### Advantageous Effects of Invention

A method for producing a pluripotent stem cell according to the present invention makes it possible to obtain a pluripotent stem cell that maintains an undifferentiated state, and exhibits excellent efficiency of differentiation when the pluripotent stem cell population is produced by suspension culture.

In addition, a method for inducing differentiation of a pluripotent stem cell according to the present invention can achieve excellent efficiency of differentiation of the pluripotent stem cell that maintains an undifferentiated state.

### Brief Description of Drawings

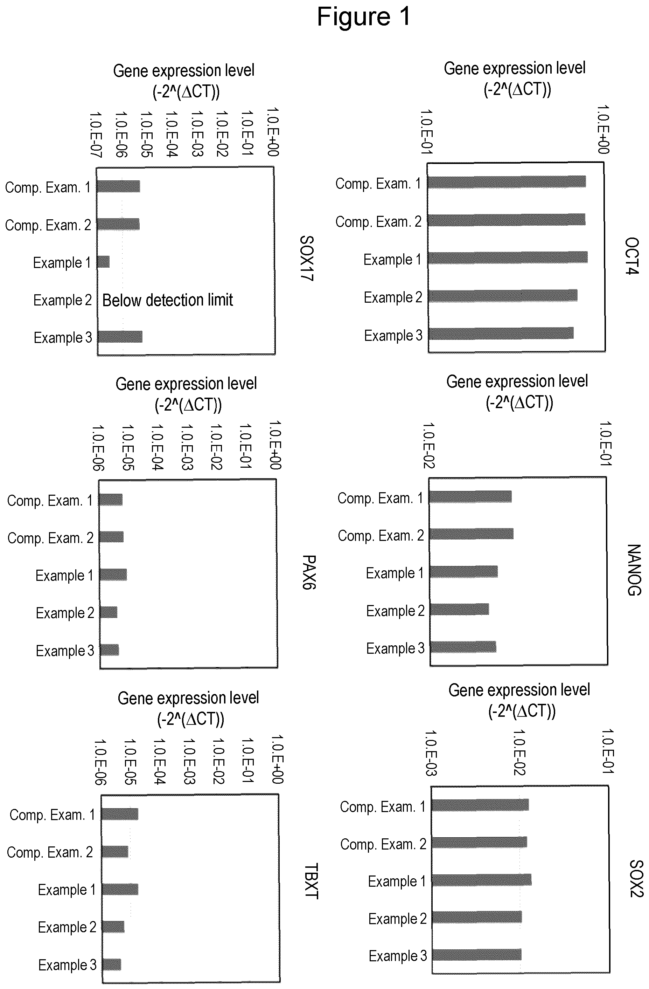

[Figure 1] Figure 1 is characteristic graphs illustrating the expression levels of undifferentiation markers (OCT4, NANOG, and SOX2) and the expression levels of differentiation markers (TBXT, SOX17, and PAX6), in which the expression levels were measured, by quantitative real-time PCR, using the cells cultured by suspension culture in Comparative Examples 1 and 2 and Examples 1, 2, and 3.

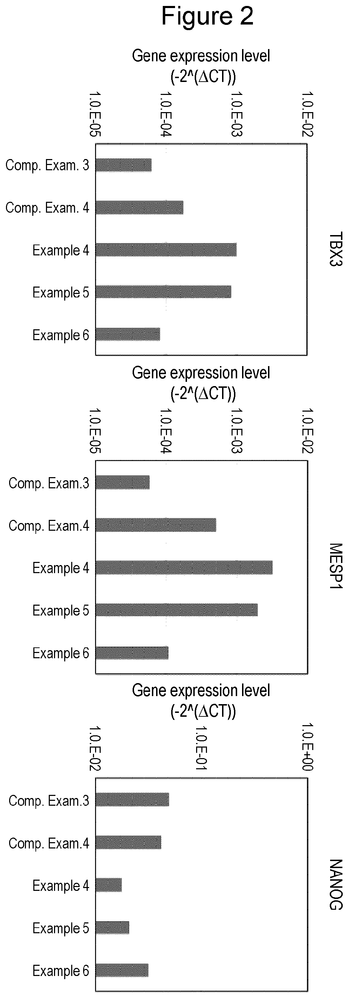

[Figure 2] Figure 2 is characteristic graphs illustrating the expression levels of differentiation markers (TBX3 and MESP1) and the expression levels of undifferentiation markers (NANOG), in which the expression levels were measured, by quantitative real-time PCR, using the cells obtained by inducing differentiation in Comparative Examples 3 and 4 and Examples 4, 5, and 6.

### Description of Embodiments

### 1. Method for Producing Pluripotent Stem Cell

### 1-1. Outline

A method for producing a pluripotent stem cell according to the present invention is characterized by obtaining a pluripotent stem cell or a pluripotent stem cell population that maintains an undifferentiated state, and has excellent efficiency of differentiation, wherein the pluripotent stem cell or the pluripotent stem cell population is obtained by suspension-culturing the pluripotent stem cell in the presence of a protein kinase Cβ (PKCβ) inhibitor and a WNT inhibitor, followed by suspension-culturing the pluripotent stem cell in a medium not substantially containing a PKCβ inhibitor and/or a WNT inhibitor. Accordingly, differentiation can be induced efficiently by using a pluripotent stem cell obtained by a method for producing a pluripotent stem cell according to the present invention.

### 1-2. Definitions of Terms

The following terms used herein are defined.

### <<Cell>>

A "pluripotent stem cell" as a subject of the invention herein refers to a cell that has multipotency (pluripotency) capable of differentiating the cell into all kinds of cells constituting a living body, and can continue to grow infinitely in in vitro culture under suitable conditions, simultaneously maintaining the pluripotency. More specifically, pluripotency means the capability to differentiate into a germ layer constituting an individual (in the case of a vertebrate, the germ layer is one of the three germ layers, i.e., an ectoderm, mesoderm, and endoderm or a precursor cell common between an endoderm and a mesoderm, and referred to as a mesendoderm). Examples of such a cell include an embryonic stem cell (ES cell), embryonic germ cell (EG cell), germline stem cell (GS cell), and induced pluripotent stem cell (iPS cell). An "ES cell" is a pluripotent stem cell prepared from an early embryo. An "EG cell" is a pluripotent stem cell prepared from a fetal primordial germ cell (Shamblott M. J. et al., 1998, Proc. Natl. Acad. Sci. USA., 95:13726-13731). A "GS cell" is a pluripotent stem cell prepared from a cell testis (Conrad S., 2008, Nature, 456:344-349). In addition, an "iPS cell" refers to a pluripotent stem cell obtained by reprogramming by which a small number of genes encoding reprogramming factors are introduced into a differentiated somatic cell to render the somatic cell undifferentiated.

A pluripotent stem cell herein may be a cell derived from a multicellular organism. The pluripotent stem cell is preferably an animal-derived cell, more preferably a mammal-derived cell. Examples thereof include: rodents such as mice, rats, hamsters, and guinea pigs; livestock or pet animals such as dogs, cats, rabbits, bovines, horses, sheep, and goats; and primates such as humans, rhesus monkeys, gorillas, and chimpanzees. Human-derived cells are particularly preferable.

The pluripotent stem cell used herein includes a naive pluripotent stem cell and a primed pluripotent stem cell. A naive pluripotent stem cell is defined as a cell in a near-pluripotent state found in the pre-implantation inner cell mass. A primed pluripotent stem cell is defined as a cell in a near-pluripotent state found in the post-implantation epiblast. A primed pluripotent stem cell is characterized in that, compared to a naive pluripotent stem cell, the primed pluripotent stem cell has a low chimera-forming ability, has only one transcriptionally active X chromosome, and has a higher level of transcriptionally repressive histone modification. In addition, the OTX2 gene is a marker gene for a primed pluripotent stem cell, and the REX1 gene and KLF family gene are naive pluripotent stem cell marker genes. Furthermore, a colony formed by primed pluripotent stem cells is in oblate shape, and a colony formed by naive pluripotent stem cells is in dome shape. A pluripotent stem cell to be used herein is preferably a primed pluripotent stem cell in particular.

A pluripotent stem cell to be used herein may be a commercially available cell, a distributed cell, or a newly prepared cell. In this regard, the pluripotent stem cell to be used in each invention described herein is preferably an iPS cell or an ES cell, without limitation.

Examples of a commercially available cell line that can be used as an iPS cell herein include, but are not limited to: 253G1, 253G4, 201B6, 201B7, 409B2, 454E2, 606A1, 610B1, 648A1, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC38-2, MSC-iPSC1, BJ-iPSC1, RPChiPS771-2, WTC-11, 1231A3, 1383D2, 1383D6, 1210B2, 1201C1, and 1205B2.

In addition, examples of a clinical cell line that can be used as an iPS cell herein include, but are not limited to: QHJI01s01, QHJI01s04, QHJI14s03, QHJI14s04, Ff-l14s03, Ff-l14s04, and YZWI.

In addition, in a case where an iPS cell used herein is a newly prepared cell, examples of a usable combination of genes of reprogramming factors to be introduced include, but are not limited to, a combination of the OCT3/4 gene, KLF4 gene, SOX2 gene, and c-Myc gene (Yu J, et al., 2007, Science, 318:1917-20) and a combination of the OCT3/4 gene, SOX2 gene, LIN28 gene, and NANOG gene (Takahashi K, et al., 2007, Cell, 131:861-72). A form in which such a gene is introduced into a cell is not particularly limited. For example, a gene may be introduced using a plasmid, a synthetic RNA may be introduced, or a gene may be introduced in protein form. In addition, an iPS cell produced by a method using a microRNA, RNA, a low-molecular-weight compound, or the like may be used, or a newly prepared clinical-grade iPS cell may be used.

Examples of a commercially available cell line that can be used as an ES cell herein include, but are not limited to, KhES-1, KhES-2, KhES-3, KhES-4, KhES-5, SEES1, SEES2, SEES3, SEES-4, SEES-5, SEES-6, SEES-7, HUES8, CyT49, H1, H9, and HS-181.

A pluripotent stem cell marker (also referred to as an undifferentiation marker) is a gene marker that is specifically or excessively expressed in a pluripotent stem cell. Examples thereof include alkaline phosphatase, NANOG, OCT4, SOX2, TRA-1-60, c-Myc, KLF4, LIN28, SSEA-4, and SSEA-1.

A pluripotent stem cell marker can be detected by any detection method in the art. Examples of a method for detecting a cell marker include, but are not limited to, flow cytometry. In a case where a fluorescence-labeled antibody is used as a detection reagent in flow cytometry, and where a cell emitting stronger fluorescence than a negative control (isotype control or FMO control) is detected, the cell can be determined to be "positive" for the marker. The percentage of cells positive for a fluorescence-labeled antibody, as analyzed by flow cytometry, is referred to as the "positive rate" in some cases. In addition, any antibody known in the art can be used as the fluorescence-labeled antibody. Examples thereof include, but are not limited to, antibodies labeled with fluorescein isothiocyanate (FITC), phycoerythrin (PE), and allophycocyanin (APC).

### <<Culture and Medium>>

"Suspension culture" is one of the cell culture methods and refers to allowing cells to grow in a suspension state in a medium. As used herein, the "suspension state" refers to a non-adherent state in which cells are not fixed to a culture container or the like in a culture solution. In addition, for example, a culture method in which cells are allowed to adhere to microcarriers, and cultured in a suspended state in a culture solution can be regarded as suspension culture. This is because, although the cells adhere to the microcarriers, a cell mass containing the microcarriers is suspended without adhering to a culture container. The "suspension culture method" is a method for culturing cells by suspension culture. Cells in this method generally exist primarily in a dissociated state at the start of the culture, and as the culture progresses, the cells exist in the form of an aggregated cell mass in the culture solution. An alternative culture method to the suspension culture method is an adherent culture method. The "adherent culture method" is a method for culturing cells in an adherent state. "Adherent culture" refers to allowing cells to adhere to an external matrix such as a culture container, and growing the cells. In this case, the external matrix or the like to which the cells are allowed to adhere is not suspended in a culture solution. In this regard, the adherent cells described above can be cultured not only by adherent culture but also by suspension culture. A suspension culture method in the present invention is preferably a suspension culture method in which no microcarrier is used.

The terms "first liquid medium" and "second liquid medium" are used herein. The first liquid medium means a liquid medium containing a PKCβ inhibitor and a WNT inhibitor, and the second liquid medium means a liquid medium not substantially containing a PKCβ inhibitor and/or a WNT inhibitor. Note that the second liquid medium may be a medium not substantially containing one of a PKCβ inhibitor and a WNT inhibitor, or may be a medium not substantially containing either inhibitor.

As used herein, a "medium" refers to a liquid or solid substance prepared to culture cells. In principle, the medium contains at least the minimum necessary amount of components essential for growth and/or maintenance of cells. Unless otherwise specified, the medium herein corresponds to a liquid medium that is for animal cells, and is to be used to culture animal-derived cells.

As used herein, the "basal medium" refers to a medium serving as the basis for various media for animal cells. Culture is possible with a basal medium alone. It is also possible to prepare a medium according to the purpose, for example, to prepare a medium specific to one kind of cell by adding any kind of culture additive. Examples of the basal medium that can be used herein include, but are not particularly limited to, BME medium, BGJb medium, CMRL1066 medium, Glasgow GMEM, Improved MEM Zinc Option medium, IMDM (Iscove's Modified Dulbecco's Medium), Medium 199, Eagle MEM Medium, αMEM Medium, DMEM (Dulbecco's Modified Eagle's Medium), Ham's F10 medium, Ham's F12 medium, RPMI 1640 medium, Fischer's medium, and a medium mixture thereof (e.g., DMEM/F12 medium (Dulbecco's Modified Eagle's Medium/Nutrient Mixture F-12 Ham)). As the DMEM/F12 medium, in particular, a medium obtained by mixing a DMEM medium and a Ham's F12 medium at a weight ratio in the range of preferably 60/40 to 40/60, for example, 58/42, 55/45, 52/48, 50/50, 48/52, 45/55, or 42/58 is used. Another medium, such as used for culturing a human iPS cell and a human ES cell, can also be suitably used.

A medium to be used in the present invention is preferably a medium that does not contain serum, i.e., a serum-free medium.

As used herein, a "culture additive" is a substance that is other than serum, and is to be added to a medium for the purpose of culture. Specific examples of the culture additive include, but are not limited to, L-ascorbic acid, insulin, transferrin, selenium, sodium hydrogen carbonate, growth factors, fatty acids or lipids, amino acids (e.g., non-essential amino acids), vitamins, cytokines, antioxidants, 2-mercaptoethanol, pyruvic acid, buffers, inorganic salts, and antibiotics. Insulin, transferrin, and cytokines may be naturally occurring proteins isolated, for example, from tissues or serum of animals (preferably humans, mice, rats, bovines, horses, goats, and the like), or may be genetically engineered recombinant proteins. In addition, examples of the growth factor that can be used include, but are not limited to, basic fibroblast growth factor-2 (FGF2), transforming growth factor-β1 (TGF-β1), Activin A, IGF-1, MCP-1, IL-6, PAI, PEDF, IGFBP-2, LIF, and IGFBP-7. Examples of the antibiotic that can be used include, but are not limited to, penicillin, streptomycin, and amphotericin B. A particularly preferable growth factor as a culture additive for a medium to be used in the present invention is FGF2 and/or TGF-β1.

In addition, the medium preferably contains a ROCK inhibitor. Examples of the ROCK inhibitor include Y-27632. Containing a ROCK inhibitor in the medium makes it possible to significantly suppress cell death in the suspension culture of a pluripotent stem cell, and increase the strength of a cell mass so that the resistance to physical damage can be improved. For example, the lower limit of the concentration of the ROCK inhibitor in the medium can be 1 µM, 2 µM, 3 µM, 5 µM, 7 µM, or 10 µM, and the upper limit can be 50 µM, 40 µM, 30 µM, 20 µM, or 10 µM.

Furthermore, the composition of the medium preferably contains no LIF particularly in a case where the medium is used to culture a primed pluripotent stem cell. Furthermore, it is preferable that the composition of the medium does not contain one of, preferably any of, a GSK3 inhibitor and an MEK/ERK inhibitor in a case where the medium is used to culture a primed pluripotent stem cell. In a medium containing none of these LIF, GSK3 inhibitor, and MEK/ERK inhibitor, a primed pluripotent stem cell can be cultured, maintaining an undifferentiated state, without becoming naive.

A medium to be used in the present invention may contain one or more culture additives described above. In general, examples of a medium to be supplemented with the culture additives include, but are not limited to, the basal medium.

A culture additive to be added to the medium can be in the form of a solution, a derivative, a salt, a reagent mixture, or the like. For example, L-ascorbic acid to be added to the medium may be in the form of a derivative such as magnesium ascorbate 2-phosphate. Selenium to be added to the medium may be in the form of a selenite salt (for example, sodium selenite). In addition, insulin, transferrin, and selenium to be added to the medium may be in the form of an insulin-transferrin-selenium (ITS) reagent. In addition, a commercially available medium supplemented with at least one selected from L-ascorbic acid, insulin, transferrin, selenium, or sodium hydrogen carbonate may also be used. Examples of the commercially available medium supplemented with insulin and transferrin include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE^{™} (BioWhittaker), UltraDOMA^{™} (BioWhittaker), UltraCHO^{™} (BioWhittaker), UltraMDCK^{™} (BioWhittaker), STEMPRO (registered trademark) hESC SFM (Life Technologies Japan Ltd.), Essential 8^{™} (Life Technologies Japan Ltd.), StemFit (registered trademark) AK02N (Ajinomoto Co., Inc.), mTeSR1 (Veritas), TeSR2 (Veritas), ReproMed (Reprocell Inc.), and StemScale (Thermo Fisher Scientific Inc.).

A medium to be most preferably used in the present invention is a serum-free medium containing: L-ascorbic acid, insulin, transferrin, selenium, and sodium hydrogen carbonate; and at least one growth factor. A serum-free DMEM/F12 medium containing L-ascorbic acid, insulin, transferrin, selenium, sodium hydrogen carbonate, and at least one growth factor (preferably FGF2 and TGF-β1) is particularly preferable.

### <<PKCβ Inhibitor>>

As used herein, a "protein kinase Cβ (PKCβ) inhibitor" refers to a substance that inhibits or suppresses the activity of PKCβ. A protein kinase has a C-terminal catalytic region and an N-terminal regulatory region. The catalytic region is composed of a sequence that recognizes a phosphorylated residue on a substrate protein and a sequence that forms an ATP-Mg²⁺ binding site. The regulatory region is composed of a C1 domain and a C2 domain.

PKC encompasses PKCα, PKCβ I, PKCβ II, and PKCγ as conventional isozymes. In addition, PKC encompasses novel isozymes such as PKCδ, PKCε, PKCθ, and PKCη, and atypical isozymes such as PKCζ, PKCλ, and PKCµ.

As used herein, "PKCβ" encompasses both PKCβ I and PKCβ II, or encompasses one of PKCβ I and PKCβ II. As used herein, the "PKCβ inhibitor" refers to a substance that inhibits at least PKCβ I and/or PKCβ II among these conventional, novel, and atypical isozymes. That is, the PKCβ inhibitor encompasses a substance that inhibits or suppresses only the activity of PKCβ I, a substance that inhibits or suppresses only the activity of PKCβ II, and a substance that inhibits or suppresses the activity of each of PKCβ I and PKCβ II.

The PKCβ inhibitor may be a substance that specifically inhibits or suppresses only the activity of PKCβ, but may also be a substance that inhibits or suppresses the activity of an isozyme other than PKCβ I or PKCβ II. For example, the PKCβ inhibitor may be a substance that inhibits or suppresses the activity of any of the above-described conventional, novel, and atypical isozymes encompassing PKCβ I and PKCβ II. In addition, the PKCβ inhibitor may be a substance that inhibits or suppresses the activity of any of the conventional isozymes PKCα and PKCγ in addition to PKCβ I and PKCβ II. Furthermore, the PKCβ inhibitor may be a substance that inhibits or suppresses the activity of any of the novel isozymes PKCδ, PKCε, PKCθ, and PKCη in addition to PKCβ I and PKCβ II.

Examples of the above-described PKCβ inhibitors include: a compound that acts directly or indirectly on PKCβ; an antisense nucleic acid against the gene encoding PKCβ; an RNA interference-inducing nucleic acid (for example, siRNA); a dominant-negative mutant; and an expression vector thereof.

By way of example, the PKCβ inhibitor may include a compound having the following structural formula [Formula I].

A compound represented by the following Formula I or a salt thereof:

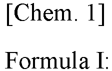

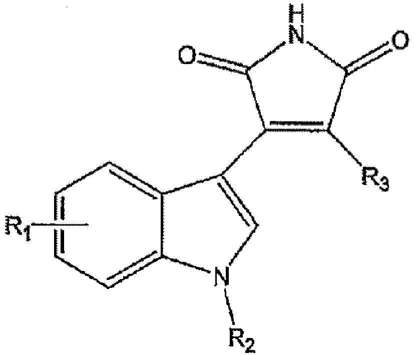

In Formula I,
R₁ is a hydrogen atom or a C₁₋₃ alkoxy group (preferably a methoxy group), R₂ is a hydrogen atom, a C₁₋₃ alkyl group (preferably a methyl group), or a -N(R_{A})₂-substituted C₁₋₃ alkyl group (preferably -(CH₂)₃-N(CH₃)₂);
R_{A} is independently an ethyl group or a methyl group (preferably a methyl group);
R₃ is a group represented by 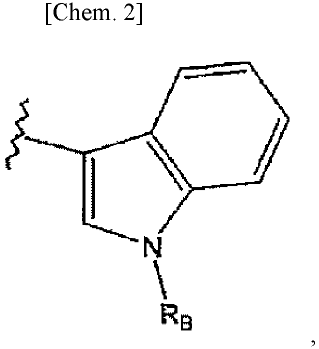 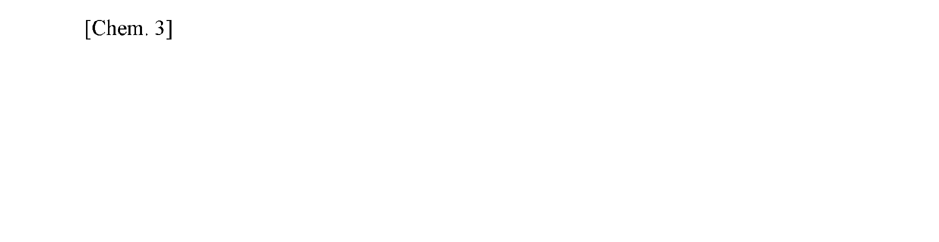 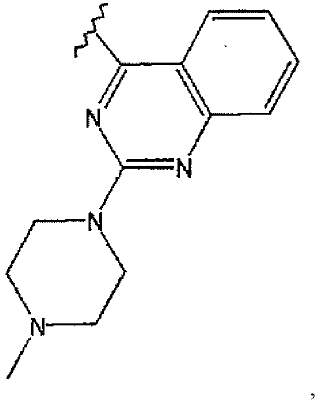 or 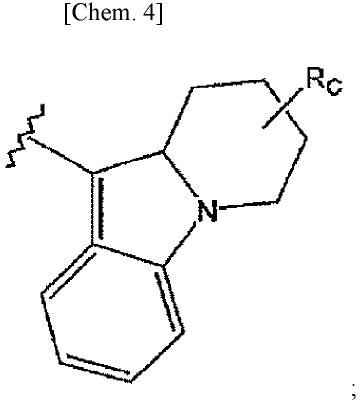
R_{B} is a hydrogen atom, -S-C(=NH)(-NH₂)-substituted C₂₋₄ alkyl group (preferably - (CH₂)₃-S-C(=NH)(-NH₂)), or 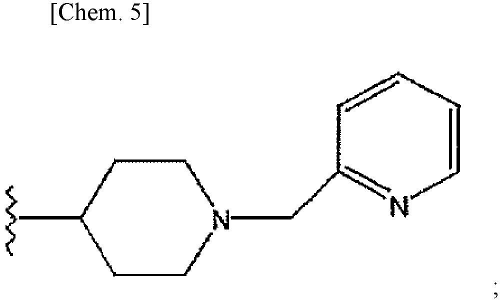
or R₂ and R_{B} together may form the following divalent group: 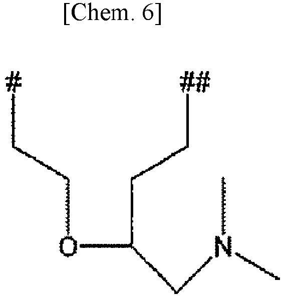
wherein "#" represents the binding to the binding position of R₂, "##" represents the binding to the binding position of R_{B}; and
the steric configuration of the asymmetric carbon contained in the divalent group is not particularly limited, but is preferably 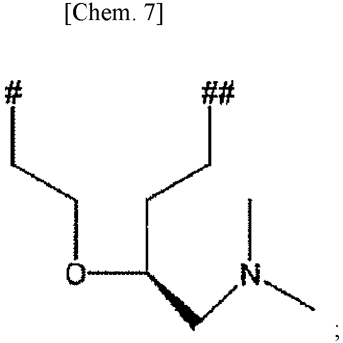
R_{C} is a -N(R_{D})₂-substituted C₁₋₃ alkyl group (preferably -(CH₂)-N(CH₃)₂); and
R_{D} is independently an ethyl group or a methyl group (preferably a methyl group).

Examples of the salt of the compound represented by Formula I include hydrochloride and sulfate.

Specific examples of the PKCβ inhibitor having the above-described structural formula [Formula I] include a compound selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo-Dihydrosphingosine, and Melittin. It is particularly preferable to use a compound selected from the group consisting of Go6983, GF109203X, and LY-333531 among the PKCβ inhibitors having the above-described structural formula.

The structural formula of Go6983 (3-[1-[3-(dimethylamino)propyl]-5-methoxy-1H-indole-3-yl]-4-(1*H*-indole-3-yl)-1*H*-pyrrole-2,5-dione) is shown below.

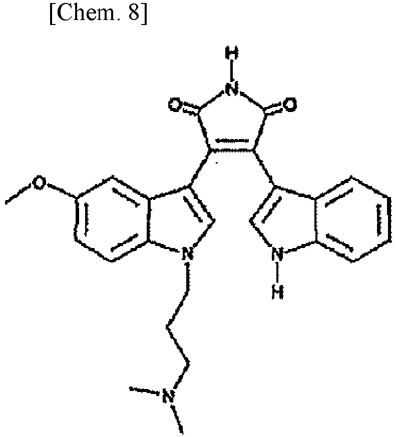

The structural formula of GF109203X (2-[1-(3-dimethylaminopropyl)indole-3-yl]-3-(indole-3-yl)maleimide) is shown below.

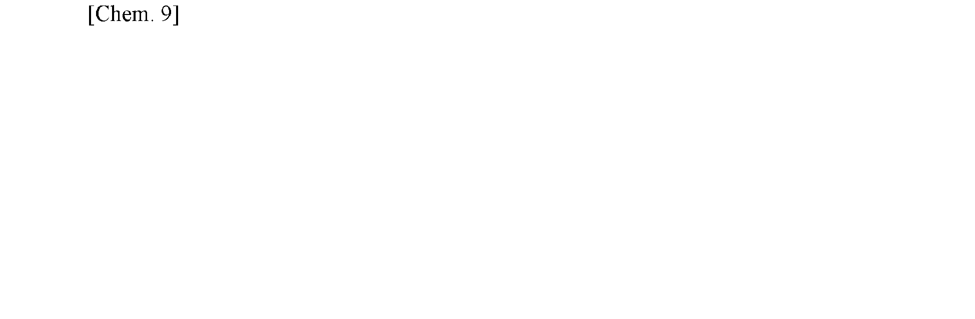

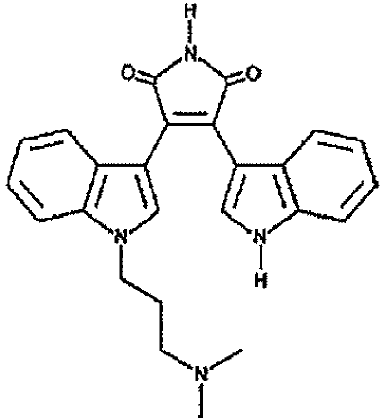

The structural formula of LY-333531((9S)-[(dimethylamino)methyl]-6,7,10,11-tetrahydro-9*H*,18*H*-5,21:12,17-dimethenodibenzo[e,k]pyrrolo[3,4-h][1,4,13]oxadiazacyclohexadecine-18,20(19*H*)-dione, monohydroxy chloride) is shown below.

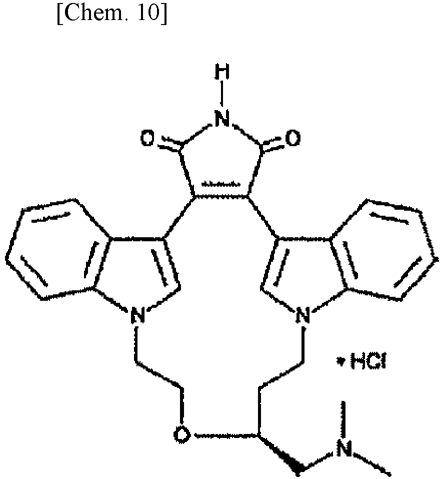

The structural formula of enzastaurin (3-(1-methylindole-3-yl)-4-[1-[1-(pyridine-2-ylmethyl)piperidine-4-yl]indole-3-yl]pyrrole-2,5-dione) is shown below.

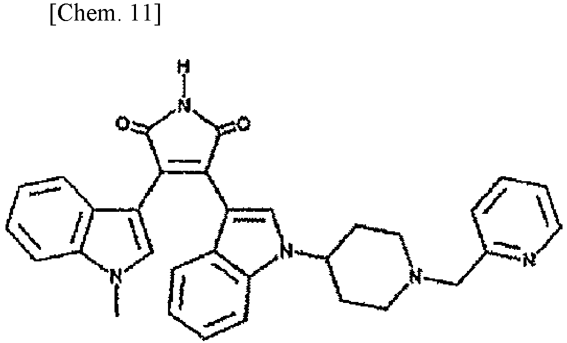

The structural formula of sotrastaurin (3-(1*H*-indole-3-yl)-4-(2-(4-methylpiperazine-1-yl)quinazoline-4-yl)-1*H*-pyrrole-2,5-dione) is shown below.

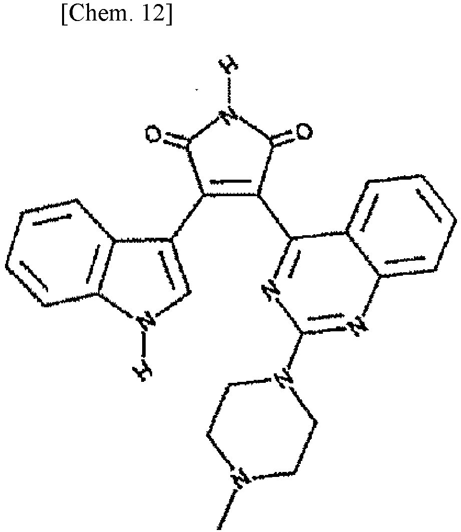

The structural formula of Ro-31-8220-mesylate (3-[3-[2,5-dihydro-4-(1-methyl-1*H-*indole-3-yl)-2,5-dioxo-1*H*-pyrrole-3-yl]-1*H*-indole-1-yl]propyl carbamimidic thio acid ester mesylate) is shown below.

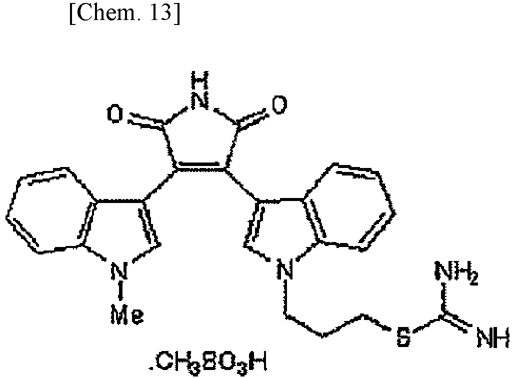

The structural formula of Ro-32-0432-hydrochloride (3-[(8S)-8-[(dimethylamino)methyl]-6,7,8,9-tetrahydropyrido[1,2-a]indole-10-yl]-4-(1-methyl-1*H-*indole-3-yl)-1*H*-pyrrole-2,5-dione hydrochloride) is shown below.

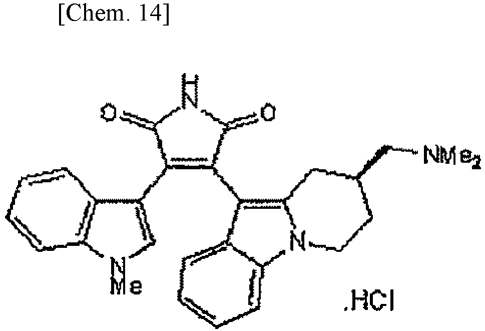

### <<WNT Inhibitor>>

As used herein, the "WNT signal (WNT) inhibitor" encompasses all substances that inhibit or reduce WNT signals. WNT is a secretory intercellular signaling protein, and is known to be involved in intracellular signaling. The signaling pathway involving WNT controls, for example, cell growth, differentiation, and movement, as well as body axis formation and organ formation during early embryonic development. The signal pathway involving WNT includes several different intracellular signaling mechanisms that are activated by WNT acting on cells. The signal pathway involving WNT includes the β-catenin pathway that controls gene expression via β-catenin, the planar cell polarity (PCP) pathway that controls the planar cell polarity of cells, and the Ca²⁺ pathway that promotes intracellular Ca²⁺ mobilization. The WNT inhibitor herein may inhibit any pathway included in the signaling pathway involving WNT.

Examples of the WNT inhibitor include proteins such as secretory Frizzled-related protein, Dickkopf (DKK) protein, Sclerostin, Cerberus, and Kremen. Specific examples of the WNT inhibitor include, but are not particularly limited to, IWR-1-endo, IWP-2, IWP-3, WNT-C59, XAV-939, CCT251545, KY1220, iCRT14, iCRT3, LF3, PNU-74654, KYA1797K, Capmatinib (INCB28060), KY02111, RCM-1, Resibufogenin, MSAB, PRI-724, Tegatrabetan (BC-2059), JW55, Adavivint (SM04690), Triptonide, Isoquercitrin, IQ-1, Salinomycin, and FH535. Further examples of the WNT inhibitor include WNT receptor inhibitors, soluble WNT receptors, WNT antibodies, casein kinase inhibitors, and dominant-negative WNT proteins.

These WNT inhibitors encompass what is called a tankyrase (TNKS) inhibitor. In the present invention, it is particularly preferable to use a TNKS inhibitor as the WNT inhibitor.

### <<TNKS Inhibitor>>

As used herein, the "tankyrase (TNKS) inhibitor" refers to a substance that inhibits or suppresses the activity of tankyrase. Tankyrase belongs to the poly(ADP-ribosyl)transferase (PARP) family, which poly(ADP-ribosyl)ates target proteins. Tankyrase-1 (tankyrase-1/PARP-5a) and tankyrase-2 (tankyrase-2/PARP-5b) are known. Tankyrase is known to have the function of promoting telomere elongation due to telomerase, by modifying the telomere protein TRF1 with poly(ADP-ribosyl), and releasing the TRF1 from telomere.

TNKS as used herein encompasses both tankyrase 1 and tankyrase 2 or encompasses one of tankyrase 1 and tankyrase 2. As used herein, the TNKS inhibitor refers to a substance that inhibits tankyrase 1 and/or tankyrase 2. That is, the TNKS inhibitor encompasses a substance that inhibits or suppresses only the activity of tankyrase 1, a substance that inhibits or suppresses only the activity of tankyrase 2, and a substance that inhibits or suppresses the activity of each of tankyrase 1 and tankyrase 2.

Examples of the TNKS inhibitor include: a compound that acts directly or indirectly on TNKS; an antisense nucleic acid against the gene encoding TNKS; an RNA interference-inducing nucleic acid (e.g., siRNA); a dominant-negative mutant; and an expression vector thereof.

By way of example, the TNKS inhibitor may be a compound selected from the group consisting of IWR-1-endo, XAV939, G007-LK, G244-LM, MSC2504877, and WIKI4. It is particularly preferable to use IWR-1-endo and/or XAV939 among the TNKS inhibitors.

The structural formula of IWR-1-endo (4-(1,3,3a,4,7,7a-hexahydro-1,3-dioxo-4,7-methano-2H-isoindole-2-yl)-N-8-quinolinyl-benzamide) is shown below.

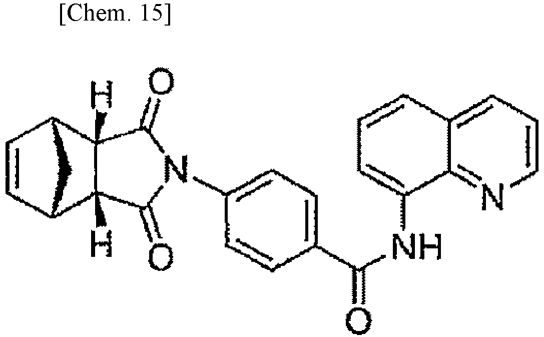

The structural formula of XAV939 (3,5,7,8-tetrahydro-2-[4-(trifluoromethyl)phenyl]-4*H*-thiopyrano[4,3-d]pyrimidine-4-one) is shown below.

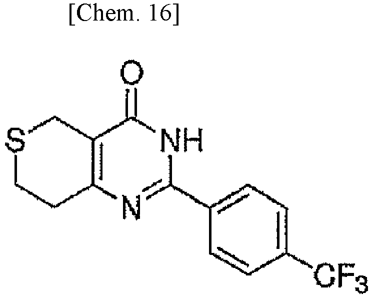

The structural formula of G007-LK (4-[5-[(1E)-2-[4-(2-chlorophenyl)-5-[5-(methylsulfonyl)-2-pyridinyl]-4*H*-1,2,4-thiazol-3-yl]ethenyl]-1,3,4-oxadiazole-2-yl]-benzonitrile) is shown below.

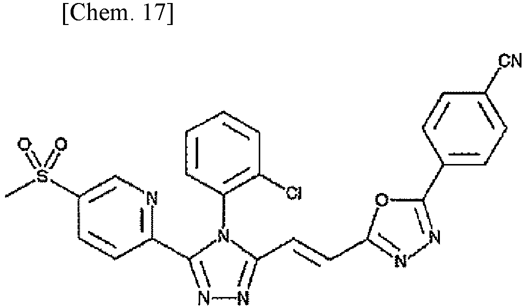

The structural formula of G244-LM (3,5,7,8-tetrahydro-2-[4-[2-(methylsulfonyl)phenyl]-1-piperazinyl]-4*H*-thiopyrano[4,3-d]pyrimidine-4-one) is shown below.

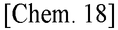

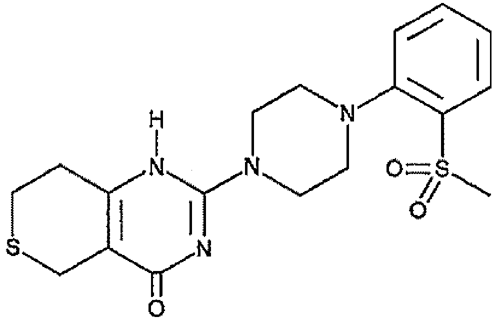

The structural formula of WIKI4 (2-[3-[[4-(4-methoxyphenyl)-5-(4-pyridinyl)-4*H-*1,2,4-thiazol-3-yl]thio]propyl]-1*H*-benzo[de]isoquinoline-1,3(2*H*)-dione) is shown below.

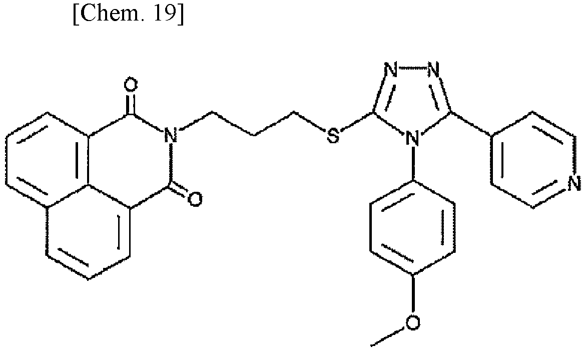

### 1-3. Constitution

### 1-3-1. First Liquid Medium

The first liquid medium used in a method for producing a pluripotent stem cell according to the present invention contains a PKCβ inhibitor and a WNT inhibitor as defined in 1-2 above. Note that the first liquid medium can also be prepared using a pluripotent stem cell differentiation inhibitor containing a PKCβ inhibitor and a WNT inhibitor as active ingredients.

Here, the first liquid medium may contain one kind of PKCβ inhibitor or a combination of two or more different kinds thereof.

The lower limit of the concentration of the PKCβ inhibitor is not particularly limited, and may be determined depending on the range in which the PKCβ inhibitor has a differentiation-suppressing effect on a pluripotent stem cell. For example, the final concentration of the PKCβ inhibitor in the first liquid medium can be 25 nM or more, 30 nM or more, 50 nM or more, 80 nM or more, 100 nM or more, 150 nM or more, 200 nM or more, 500 nM or more, or 700 nM or more.

The upper limit of the concentration of the PKCβ inhibitor is not particularly limited, and can be determined depending on the range in which the PKCβ inhibitor has a differentiation-suppressing effect on a pluripotent stem cell, the range in which the PKCβ inhibitor does not exhibit toxicity to a pluripotent stem cell, the solubility of the PKCβ inhibitor, and the like. For example, the final concentration of the PKCβ inhibitor in the first liquid medium can be 15 µM or less, 10 µM or less, 5 µM or less, 3 µM or less, or 1 µM or less.

On the other hand, the first liquid medium may contain one kind of WNT inhibitor or a combination of two or more different kinds thereof.

The lower limit of the concentration of the WNT inhibitor is not particularly limited, and can be determined depending on the range in which the WNT inhibitor has a differentiation-suppressing effect on a pluripotent stem cell. For example, the final concentration of the WNT inhibitor in the first liquid medium can be 90 nM or more, 100 nM or more, 150 nM or more, 200 nM or more, 300 nM or more, 400 nM or more, 500 nM or more, 600 nM or more, 700 nM or more, 800 nM or more, or 900 nM or more.

The upper limit of the concentration of the WNT inhibitor is not particularly limited, and can be determined depending on the range in which the WNT inhibitor has a differentiation-suppressing effect on a pluripotent stem cell, the range in which the WNT inhibitor does not exhibit toxicity to a pluripotent stem cell, the solubility of the WNT inhibitor, and the like. For example, the final concentration of the WNT inhibitor in the first liquid medium can be 40 µM or less, 35 µM or less, 30 µM or less, 15 µM or less, 10 µM or less, 5 µM or less, 3 µM or less, 1.5 µM or less, or 1 µM or less.

In addition, the lower limit of the concentration (molar concentration) ratio of the PKCβ inhibitor and the WNT inhibitor in the first liquid medium is not particularly limited, but can be, for example, 167:1 or more, 111:1 or more, 56:1 or more, 33:1 or more, 11:1 or more, 7.8:1 or more, 5.6:1 or more, 2.2:1 or more, 1.7:1 or more, or 1.1:1 or more. The upper limit of the concentration (molar concentration) ratio of the PKCβ inhibitor and the WNT inhibitor in the first liquid medium is not particularly limited, but can be, for example, 1:1600 or less, 1:1400 or less, 1:1200 or less, 1:600 or less, 1:400 or less, 1:200 or less, 1:120 or less, 1:60 or less, 1:40 or less, 1:36 or less, 1:32 or less, 1:28 or less, 1:24 or less, 1:20 or less, 1:16 or less, 1:12 or less, 1:8 or less, 1:6 or less, or 1:4 or less. The concentration (molar concentration) ratio of the PKCβ inhibitor and the WNT inhibitor in the first liquid medium is not particularly limited but can be, for example, in the range of 167:1 or more and 1:1600 or less. In addition, the concentration (molar concentration) ratio of the PKCβ inhibitor and the WNT inhibitor in the first liquid medium can be in the range of preferably 111:1 or more and 1:1600 or less, 56:1 or more and 1:1600 or less, 33:1 or more and 1:1600 or less, 11:1 or more and 1:1600 or less, 7.8:1 or more and 1:1600 or less, 5.6:1 or more and 1:1600 or less, 2.2:1 or more and 1:1600 or less, 1.7:1 or more and 1:1600 or less, or 1.1:1 or more and 1:1600 or less. Furthermore, the concentration (molar concentration) ratio of the PKCβ inhibitor and the WNT inhibitor in the first liquid medium can be in the range of 167:1 or more and 1:1400 or less, 167:1 or more and 1:1200 or less, 167:1 or more and 1:600 or less, 167:1 or more and 1:400 or less, 167:1 or more and 1:200 or less, 167:1 or more and 1:120 or less, 167:1 or more and 1:60 or less, 167:1 or more and 1:40 or less, 167:1 or more and 1:36 or less, 167:1 or more and 1:32 or less, 167:1 or more and 1:28 or less, 167:1 or more and 1:24 or less, 167:1 or more and 1:20 or less, 167:1 or more and 1:16 or less, 167:1 or more and 1:12 or less, 167:1 or more and 1:8 or less, 167:1 or more and 1:6 or less, or 167:1 or more and 1:4 or less.

In addition, the PKCβ inhibitor and the WNT inhibitor as active ingredients can also be added to a medium in combination with a support. The support encompasses a solvent and/or an excipient.

Examples of the solvent include water, buffers (including PBS), physiological saline, and organic solvents (DMSO, DMF, xylene, and lower alcohols).

Examples of the excipient include antibiotics, buffers, thickeners, colorants, stabilizers, surfactants, emulsifiers, antiseptics, preservatives, and antioxidants. Examples of the antibiotic that can be used include, but are not particularly limited to, penicillin, streptomycin, and amphotericin B. Examples of the buffer include phosphate buffers, tris-hydrochloric acid buffers, and glycine buffers. Examples of the thickener include gelatin and polysaccharides. Examples of the colorant include phenol red. Examples of the stabilizer include albumin, dextran, methyl cellulose, and gelatin. Examples of the surfactant include cholesterol, alkyl glycoside, alkyl polyglucoside, alkyl monoglyceryl ether, glucoside, maltoside, neopentyl glycol type, polyoxyethylene glycol type, thioglucoside, thiomaltoside, peptide, saponin, phospholipid, fatty acid sorbitan ester, and fatty acid diethanolamide. Examples of the emulsifier include glycerin fatty acid ester, sorbitan fatty acid ester, propylene glycol fatty acid ester, and sucrose fatty acid ester. Examples of the antiseptic include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white sugar, honey, isobutyl paraoxybenzoate, isopropyl paraoxybenzoate, ethyl paraoxybenzoate, butyl paraoxybenzoate, propyl paraoxybenzoate, methyl paraoxybenzoate, l-menthol, and eucalyptus oil. Examples of the preservative include benzoic acid, sodium benzoate, ethanol, sodium edetate, dry sodium sulfite, citric acid, glycerin, salicylic acid, sodium salicylate, dibutylhydroxytoluene, D-sorbitol, sorbic acid, potassium sorbate, sodium dehydroacetate, isobutyl paraoxybenzoate, isopropyl paraoxybenzoate, ethyl paraoxybenzoate, butyl paraoxybenzoate, propyl paraoxybenzoate, methyl paraoxybenzoate, propylene glycol, and phosphoric acid. Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, superoxide dismutase (SOD), glutathione peroxidase, glutathione-S-transferase, glutathione reductase, catalase, ascorbate peroxidase, and mixtures thereof.

In addition, the first liquid medium and the second liquid medium may contain one or more growth factors. Examples of the growth factor include FGF2 and TGF-β1.

### 1-3-2. Second Liquid Medium

In a method for producing a pluripotent stem cell according to the present invention, a pluripotent stem cell is cultured in a liquid medium (second liquid medium) not substantially containing a PKCβ inhibitor and/or a WNT inhibitor after the cell is cultured using the first liquid medium.

The second liquid medium is a liquid medium that differs from the first liquid medium described above in that the second liquid medium does not substantially contain a PKCβ inhibitor and/or a WNT inhibitor. Herein, the condition that the second liquid medium does not substantially contain a PKCβ inhibitor and/or a WNT inhibitor is not limited to a case where the medium does not at all contain a PKCβ inhibitor and/or a WNT inhibitor or to a case where the medium contains a PKCβ inhibitor and/or a WNT inhibitor at or below the detection limit, but the condition encompasses a case where the concentration of a PKCβ inhibitor and/or a WNT inhibitor is lower than the concentration at which the inhibitor(s) exhibit(s) the effect of suppressing differentiation of a pluripotent stem cell.

A concentration lower than the concentration at which the PKCβ inhibitor exhibits the effect of suppressing differentiation of a pluripotent stem cell depends on the kind of a pluripotent stem cell, the composition of another medium, and the culture condition, and is, for example, construed to be inclusive of a final concentration of less than 25 nM. In other words, the second liquid medium can contain the PKCβ inhibitor at a final concentration of less than 25 nM, less than 23 nM, less than 20 nM, less than 15 nM, less than 10 nM, less than 5 nM, or less than 3 nM. Most preferably, the second liquid medium contains a PKCβ inhibitor at or below the detection limit.

A concentration lower than the concentration at which the WNT inhibitor exhibits the effect of suppressing differentiation of a pluripotent stem cell depends on the kind of a pluripotent stem cell, the composition of another medium, and the culture condition, and is, for example, construed to be inclusive of a case where the final concentration is less than 90 nM. In other words, the second liquid medium can contain the WNT inhibitor at a final concentration of less than 90 nM, less than 85 nM, less than 80 nM, less than 75 nM, less than 70 M, less than 65 nM, less than 60 nM, less than 55 nM, less than 50 nM, less than 45 nM, less than 40 nM, less than 35 nM, less than 30 nM, less than 25 nM, less than 20 nM, less than 15 nM, less than 10 nM, less than 5 nM or less than 3 nM. Most preferably, the second liquid medium contains a WNT inhibitor at or below the detection limit.

Note that the second liquid medium may be a medium not substantially containing a PKCβ inhibitor and/or a WNT inhibitor, or may be a medium containing any one of a PKCβ inhibitor and a WNT inhibitor. In a case where the second liquid medium contains any one of a PKCβ inhibitor and a WNT inhibitor, the concentration of the PKCβ inhibitor can be a concentration described in the above-described item 1-3-1. First Liquid Medium.

Suspension culture using the second liquid medium cultures a pluripotent stem cell after suspension culture using the first liquid medium containing a PKCβ inhibitor and a WNT inhibitor, and there is accordingly a possibility that the PKCβ inhibitor and the WNT inhibitor contained in the first liquid medium are brought in at a stage in which the pluripotent stem cell is seeded. However, such a possibility allows one aspect in which, even if a PKCβ inhibitor and a WNT inhibitor are brought into the second liquid medium during the seeding of a pluripotent stem cell, but if the concentration of the inhibitors is within the above-described range, the pluripotent stem cell is cultured in suspension culture in the second liquid medium not substantially containing a PKCβ inhibitor and/or a WNT inhibitor.

In addition, there is a possibility that not only the PKCβ inhibitor and the WNT inhibitor but also a substance having an inhibitory effect on PKCβ and the WNT signal is produced in the medium, depending on the kind of a pluripotent stem cell cultured in the second liquid medium, the culture conditions, and the like. Even this case is encompassed in the aspect in which, as long as the concentration is within the above-described range, the pluripotent stem cell is cultured in suspension culture in the second liquid medium not substantially containing a PKCβ inhibitor and/or a WNT inhibitor.

### 1-4. Effects

A method for producing a pluripotent stem cell according to the present invention makes it possible that a pluripotent stem cell to be used for inducing differentiation is produced by culturing a pluripotent stem cell in the second liquid medium not substantially containing a PKCβ inhibitor and/or a WNT inhibitor. In such a manner, culturing a pluripotent stem cell using the second liquid medium can enhance the efficiency of differentiation of the pluripotent stem cell obtained. Accordingly, applying a method for producing a pluripotent stem cell according to the present invention makes it possible to obtain a pluripotent stem cell having excellent efficiency of differentiation.

In particular, culturing a pluripotent stem cell in the second liquid medium not substantially containing any of a PKCβ inhibitor and a WNT inhibitor makes it possible to produce a pluripotent stem cell that exhibits excellent efficiency of differentiation into any of an ectoderm, mesoderm, and endoderm. In addition, culturing a pluripotent stem cell in the second liquid medium containing a WNT inhibitor, and not substantially containing a PKCβ inhibitor makes it possible to produce a pluripotent stem cell that exhibits excellent efficiency of differentiation into an ectoderm. Furthermore, culturing a pluripotent stem cell in the second liquid medium containing a PKCβ inhibitor, and not substantially containing a WNT inhibitor makes it possible to produce a pluripotent stem cell that exhibits excellent efficiency of differentiation into a mesoderm and an endoderm.

### 2. More Detailed Description of Method for Producing Pluripotent Stem Cell

### 2-1. Outline

The step of suspension-culturing the pluripotent stem cell using the first liquid medium, the step of suspension-culturing the pluripotent stem cell using the second liquid medium, and other optional steps, included in a method for producing a pluripotent stem cell according to the present invention, will be explained below.

### 2-2. Method

The method in this aspect comprises: a suspension culture step using the first liquid medium and a suspension culture step using the second liquid medium, as essential steps; and a maintenance culture step and a collection step as optional steps.

### 2-2-1. Suspension Culture Step Using First Liquid Medium

The suspension culture step using the first liquid medium is a step in which a cell, cell population, or cell mass maintained in adherent culture or suspension culture is cultured for the purpose of growing the cell in such a manner that the undifferentiated state thereof is maintained, in which the adherent culture cultures the cell adhering to a culture substrate, such as a container or a support.

### (Culture Container)

A culture container to be used for culture is preferably a container to the inner surface of which a cell has low adhesiveness. An example of the container to the inner surface of which a cell has low adhesiveness is a plate the surface of which is treated hydrophilically with a biocompatible material. For example, Nunclon^{™} Sphera (Thermo Fisher Scientific Inc.) can be used as a culture container.

The culture container is not particularly limited to any shape, and is for example, a dish-shaped, flask-shaped, well-shaped, bag-shaped, or spinner flask-shaped culture container.

The capacity of the culture container to be used can be suitably selected, and is not particularly limited. The lower limit of the area of the bottom face of the part accommodating a culture medium, in the planar view, is preferably 0.32 cm² or more, 0.65 cm² or more, 1.9 cm² or more, 3.0 cm² or more, 3.5 cm² or more, 9.0 cm² or more, or 9.6 cm² or more, and the upper limit thereof is preferably 1000 cm² or less, 500 cm² or less, 300 cm² or less, 150 cm² or less, 75 cm² or less, 55 cm² or less, 25 cm² or less, 21 cm² or less, 10 cm² or less, or 3.5 cm² or less.

The capacity of the culture container to be used can be suitably selected, and is not particularly limited. The lower limit of the volume that can accommodate the first liquid medium and be used for culture is preferably 1 mL or more, 2 mL or more, 4 mL or more, 10 mL or more, 20 mL or more, 30 mL or more, 50 mL or more, 100 mL or more, 200 mL or more, 500 mL or more, 1 L or more, 3 L or more, 5 L or more, 10 L or more, or 20 L or more, and the upper limit thereof is preferably 100 L or less, 50 L or less, 20 L or less, 10 L or less, 5 L or less, 3 L or less, 1 L or less, 500 mL or less, 200 mL or less, 100 mL or less, 50 mL or less, or 30 mL or less.

### (Amount of Culture Solution)

The amount of the first liquid medium may be suitably adjusted, depending on a culture container to be used. Without particular limitation, these will be exemplified. For example, in a case where a 12-well plate (the area of the bottom face of the well, in the planar view, is 3.5 cm² per well) is used, the amount per well can be 0.5 mL or more, 1.5 mL or less, and preferably approximately 1.3 mL. In addition, in a case where a 6-well plate (the area of the bottom face of the well, in the planar view, is 9.6 cm² per well) is used, the lower limit of the amount per well can be 1.5 mL or more, 2 mL or more, or 3 mL or more, and the upper limit thereof can be 6 mL or less, 5 mL or less, or 4 mL or less. Furthermore, in a case where a 125 mL Erlenmeyer flask (Erlenmeyer flask having a capacity of 125 mL) is used, the lower limit of the amount per flask can be 10 mL or more, 15 mL or more, 20 mL or more, 25 mL or more, or 30 mL or more, and the upper limit thereof can be 50 mL or less, 45 mL or less, or 40 mL or less. In addition, in a case where a 500 mL Erlenmeyer flask is used, the lower limit of the amount per flask can be 100 mL or more, 105 mL or more, 110 mL or more, 115 mL or more, or 120 mL or more, and the upper limit thereof can be 150 mL or less, 145 mL or less, 140 mL or less, 135 mL or less, 130 mL or less, or 125 mL or less. Furthermore, in a case where a 1000 mL Erlenmeyer flask is used, the lower limit of the amount per flask can be 250 mL or more, 260 mL or more, 270 mL or more, 280 mL or more, or 290 mL or more, and the upper limit thereof can be 350 mL or less, 340 mL or less, 330 mL or less, 320 mL or less, or 310 mL or less. Furthermore, for example, in a case where a disposable culture bag having a capacity of 2 L is used, the lower limit of the amount per bag can be 100 mL or more, 200 mL or more, 300 mL or more, 400 mL or more, 500 mL or more, 600 mL or more, 700 mL or more, 800 mL or more, 900 mL or more, or 1000 mL or more, and the upper limit thereof can be 2000 mL or less, 1900 mL or less, 1800 mL or less, 1700 mL or less, 1600 mL or less, 1500 mL or less, 1400 mL or less, 1300 mL or less, 1200 mL or less, or 1100 mL or less. In addition, in a case where a disposable culture bag having a capacity of 10 L is used, the lower limit of the amount per bag can be 500 mL or more, 1 L or more, 2 L or more, 3 L or more, 4 L or more, or 5 L or more, and the upper limit thereof can be 10 L or less, 9 L or less, 8 L or less, 7 L or less, or 6 L or less. In addition, in a case where a single-use reactor having a capacity of 1 L is used, the lower limit of the amount per reactor can be 300 mL or more, 350 mL or more, 400 mL or more, 450 mL or more, or 500 mL or more, and the upper limit thereof can be 1 L or less, 900 mL or less, 800 mL or less, 700 mL or less, or 600 mL or less. In addition, in a case where a stirring blade-type reactor having an arbitrary capacity is used, the capacity can be within the range of the working volume specified by each manufacturer.

### (Seeding Density)

In suspension culture, the cell density (seeding density) of cells to be seeded in a new medium can be suitably adjusted, taking into consideration the culture time, the state of the cell after culture, and the number of cells required after culture. Without limitation, the lower limit thereof is usually in the range of, for example, 0.01 × 10⁵ cells/mL or more, 0.1 × 10⁵ cells/mL or more, 1 × 10⁵ cells/mL or more or 2 ×10⁵ cells/mL or more, and the upper limit thereof is in the range of, for example, 20 × 10⁵ cells/mL or less or 10 × 10⁵ cells/mL or less.

### (Culture Conditions)

Culture conditions such as culture temperature, culture time, and CO₂ concentration are not particularly limited. Culture may be performed within the range of a conventional method in the art. For example, the lower limit of the culture temperature may be 20°C or more or 35°C or more, and the upper limit thereof may be 45°C or less or 40°C or less. The culture temperature is preferably 37°C. In addition, the lower limit of the culture time in one passage period is in the range of 0.5 hours or more or 6 hours or more, and the upper limit thereof is in the range of 9 days or less, 7 days or less, 120 hours or less, 96 hours or less, 72 hours or less, or 48 hours or less. The lower limit of the CO₂ concentration during culture may be 0.5% or more, 1% or more, 2% or more, 3% or more, 4% or more, or 4.5% or more, and the upper limit thereof may be 10% or less, 5.5% or less, and preferably 5%. Note that the CO₂ concentration during culture does not need to be constant, and may be changed or varied during the culture. In addition, the culture medium can be changed at a suitable frequency. The frequency of medium change varies depending on the kind of a cell to be cultured, and may be, for example, but is not limited to, once or more in 5 days, once or more in 4 days, once or more in 3 days, once or more in 2 days, once or more in 1 day, or twice or more in 1 day. Alternatively, the medium may be changed continuously using a perfusion process. For medium change, cells may be collected in the same manner as in the collection step. Then, a fresh culture medium may be added, cell masses may be gently dispersed, and then, the cells may be cultured again. In a case where the medium is changed using a perfusion process, the cells and the medium may be separated using a filter to retain the cells in the culture system, and then the medium can be changed. The frequency and method for medium change are not limited to the frequency and method described above, and an optimal method may be suitably adopted.

In addition, the timing of terminating culture and the timing of medium change can also be determined, for example, on the basis of the lactic acid concentration of the medium. Lactic acid is produced by cells during culture, and accumulates in the medium. It is known that lactic acid produced by cells or lactic acid initially contained in the medium damages the cells, and as a result, inhibits the maintenance of the undifferentiated state of a pluripotent stem cell, and causes adverse effects such as reducing cell growth, particularly the cell growth ability after passage. In view of the above, determining the timing of culture termination and/or the timing and amount of medium change on the basis of the lactic acid concentration of the medium makes it possible to avoid the inhibitory effect of lactic acid on maintaining the undifferentiatedness and the effect of lactic acid on the reduction of the cell growth ability.

Furthermore, the final stage of the suspension culture step using the first liquid medium can be set in combination with the below-described suspension culture step using the second liquid medium. That is, the culture time necessary for the below-described suspension culture step using the second liquid medium is preliminarily set, and subtracted from the whole culture time necessary for a pluripotent stem cell as a subject of culture, thus making it possible to set the culture time in the suspension culture step using the first liquid medium.

### (Culture Method)

In this method, culture is performed in suspension culture, i.e., in a state where cells do not adhere to a container, and thus, flow culture in which the first liquid medium flows is used. The "flow culture" refers to culturing under conditions that allow the medium to flow. In the case of flow culture, a method that allows the medium to flow to promote cell aggregation is preferable. Examples of such a culture method include a rotation culture method, rocking culture method, stirring culture method, and any combination thereof.

The "rotation culture method" (including a shaking culture method) refers to a method for culturing the cells under conditions that allow a medium to flow so that cells can gather at one point owing to a stress (centrifugal force and centripetal force) caused by rotational flow. Specifically, culture is performed by rotating a culture container accommodating a medium containing cells along a substantially horizontal plane in such a manner that the culture container describes a closed orbit, such as a circle, an ellipse, an oblate circle, an oblate ellipse, or the like.

The rotation speed is not particularly limited. The lower limit thereof can be, for example, 1 rpm or more, 10 rpm or more, 50 rpm or more, 60 rpm or more, 70 rpm or more, 80 rpm or more, 83 rpm or more, 85 rpm or more, or 90 rpm or more. On the other hand, the upper limit thereof can be, for example, 200 rpm or less, 150 rpm or less, 120 rpm or less, 115 rpm or less, 110 rpm or less, 105 rpm or less, 100 rpm or less, 95 rpm or less, or 90 rpm or less. The amplitude of a shaker to be used for rotation culture is not particularly limited. The lower limit thereof can be, for example, 1 mm or more, 10 mm or more, or 20 mm or more. On the other hand, the upper limit thereof can be, for example, 200 mm or less, 100 mm or less, 50 mm or less, or 30 mm or less. The radius of rotation during rotation culture is not particularly limited. The amplitude is preferably set within the above-described range. The lower limit of the radius of rotation can be, for example, 5 mm or more or 10 mm or more, and the upper limit of the radius of rotation can be, for example, 100 mm or less or 50 mm or less. In particular, for example, in the below-described method for producing a cell mass, it is preferable to set the rotation condition within the above-described range so that a cell mass having a suitable size can be easily produced.

The "rocking culture method" refers to a method for culturing the cells under conditions that impart a rocking flow to a medium by linear reciprocating motion, such as rocking stirring. Specifically, culture is performed by rocking a culture container accommodating a medium containing cells along a plane substantially perpendicular to the horizontal plane. The rocking rate is not particularly limited. For example, given that one round trip is regarded as one time, the lower limit thereof may be 2 or more times, 4 or more times, 6 or more times, 8 or more times, or 10 or more times of rocking per minute. On the other hand, the upper limit thereof may be 50 times or less, 25 times or less, 20 times or less, or 15 times or less of rocking per minute. During rocking, it is preferable to give the culture container a slight angle, namely an inducing angle, with respect to the vertical plane. The rocking angle is not particularly limited. For example, the lower limit thereof can be 0.1° or more, 2° or more, 4° or more, 6° or more, or 8° or more, and the upper limit thereof can be 20° or less, 18° or less, 15° or less, 12° or less, or 10° or less. For example, in the below-described method for producing a cell mass, it is preferable to set the rocking condition within the above-described range so that a cell mass having a suitable size can be easily produced.

Furthermore, culture may also be performed with stirring by a combination of the above-described rotation and rocking motions.

The "stirring culture method" refers to a method for culturing in which a culture medium in a container is stirred using stirring means, such as a stirrer bar or a stirring blade, with the culture container allowed to stand still. For example, stirring culture can be achieved using a spinner flask-like culture container equipped with a stirring blade. Such a culture container is commercially available, and can also be used. In the case of a commercially available spinner flask-like culture container, the amount of cell culture composition, recommended by the manufacturer, can be suitably used. The stirring speed by the above-described stirring means is not particularly limited. The lower limit thereof can be 1 rpm or more, 10 rpm or more, 30 rpm or more, 50 rpm or more, 70 rpm or more, 90 rpm or more, 110 rpm or more, or 130 rpm or more. On the other hand, the upper limit thereof can be 200 rpm or less or 150 rpm or less.

In addition, in the "stirring culture method", it is preferable to control a shear stress applied to cells during culture. Animal cells, including pluripotent stem cells, are generally more susceptible to physical stress than other cells. Therefore, in some of the cases where excessive shear stress is applied to cells during stirring culture, the cells are physically damaged, the growth ability is reduced, and in the case of a pluripotent stem cell, the cell cannot maintain the undifferentiatedness.

In some cases, physical damage applied to cells during stirring culture is affected by local shear stress around a stirring blade. Local shear stress around a stirring blade is not limited, but is related to, for example, the blade tip speed (circumferential speed at the part where the blade diameter is the largest). The blade tip speed can be obtained as the following formula: Blade diameter [m] × Circumference ratio × Rotational speed [rps] = Blade tip speed [m/s].

Specifically, even when the blade tip speed is extremely high, such as 0.23 m/s or more, in suspension culture using the first liquid medium, stirring culture can be performed, simultaneously maintaining the undifferentiatedness of pluripotent stem cells.

In addition, the blade tip speed is not necessarily limited, and is preferably 0.05 m/s or more, preferably 0.08 m/s or more, preferably 0.10 m/s or more, preferably 0.13 m/s or more, preferably 0.17 m/s or more, preferably 0.20 m/s or more, preferably 0.23 m/s or more, preferably 0.25 m/s or more, preferably 0.30 m/s or more. Setting the blade tip speed within this range makes it possible that excessive aggregation of cells can be suppressed, and simultaneously, the undifferentiation of the pluripotent stem cells can be maintained.

Furthermore, the blade tip speed is not necessarily limited, and is preferably 1.37 m/s or less, preferably 1.00 m/s or less, preferably 0.84 m/s or less, preferably 0.50 m/s or less, preferably 0.42 m/s or less, preferably 0.34 m/s or less, preferably 0.30 m/s or less. Setting the blade tip speed within this range makes it possible that the fluid state of the medium in the culture system is stabilized, and simultaneously, the undifferentiation of the pluripotent stem cells can be maintained.

The above-described preferable range of the blade tip speed can also be applied to a single-use reactor manufactured by ABLE, which is provided for the suspension culture of pluripotent stem cells. In addition, to change the culture scale in stirring culture, the rotational speed of the stirring blade may be determined using the formula Pv = constant, without particular limitation. Pv is a per-unit-volume power required for stirring. Using the same Pv value enables stirring culture to be performed in the same manner between different scales. The formula Pv = constant can be represented as follows: Rotational speed [rpm or rps] per unit time × (Blade diameter [m])^{2/3} = Constant.

The extent to which the number of cells is increased and the state to which the cells are adjusted, in this step, may be suitably determined depending on the kind of a cell to be cultured, the purpose of cell aggregation, the kind of a medium, and the culture conditions.

### 2-2-2. Suspension Culture Step Using Second Liquid Medium

In a method for producing a pluripotent stem cell according to the present invention, the suspension culture step using the second liquid medium is a step to be performed after the above-described suspension culture step using the first liquid medium, and is a step for obtaining a pluripotent stem cell that maintains an undifferentiated state, and is to be used for differentiation-inducing treatment. In this section, the suspension culture step using the second liquid medium is described, and is basically in accordance with the culture method described in "2-2-1. Suspension Culture Step Using First Liquid Medium" above except that the second liquid medium is used. Accordingly, here, the description in common with "2-2-1. Suspension Culture Step Using First Liquid Medium" above is omitted, and only points characteristic of the suspension culture step using the second liquid medium are described in detail.

A pluripotent stem cell to be used in the present step is a pluripotent stem cell obtained in the above-described suspension culture step using the first liquid medium. The pluripotent stem cell obtained in the suspension culture step using the first liquid medium may be used in a single-cell state in the present step, or may be used in a cell-mass state in the present step. Using the second liquid medium in the present step makes it possible to obtain a pluripotent stem cell that maintains an undifferentiated state, and simultaneously has excellent efficiency of differentiation.

Whether the pluripotent stem cell maintains an undifferentiated state can be determined on the basis of the proportion (ratio) of cells in a pluripotent stem cell population obtained from the present step, in which the cells are the cells in which a pluripotent stem cell marker (also referred to as, for example, OCT4, SOX2, NANOG, or an undifferentiation marker) is expressed and/or presents positivity.

In this regard, in this step, it is possible to isolate a part of the pluripotent stem cells during suspension culture, and to verify whether the cells maintain an undifferentiated state. For example, whether the cells maintain an undifferentiated state can be verified by measuring the expression level of pluripotent stem cell markers expressed in pluripotent stem cells isolated during culture. Examples of pluripotent stem cell markers include alkaline phosphatase, NANOG, OCT4, SOX2, TRA-1-60, c-Myc, KLF4, LIN28, SSEA-4, and SSEA-1, as described above. Examples of the method for detecting these pluripotent stem cell markers include flow cytometry, as described above.

Pluripotent stem cells are isolated from the medium after termination of the present step or the medium during culture. It can be determined that any of the pluripotent stem cells maintains an undifferentiated state, in a case where the positive rate of a pluripotent stem cell marker for the pluripotent stem cell is preferably 80% or more, more preferably 90% or more, still more preferably 91% or more, still more preferably 92% or more, still more preferably 93% or more, still more preferably 94% or more, still more preferably 95% or more, still more preferably 96% or more, still more preferably 97% or more, still more preferably 98% or more, still more preferably 99% or more, or still more preferably 100%.

In addition, in the suspension culture step using the second liquid medium, whether the undifferentiated state is maintained can be verified by measuring the expression level of three germ layer markers (endodermal cell marker, mesodermal cell marker, and ectodermal cell marker) in the pluripotent stem cells isolated during culture. That is, it can be determined that the undifferentiated state is maintained, in a case where the positive rate of each of these endodermal cell marker, mesodermal cell marker, and ectodermal cell marker is preferably 20% or less, more preferably 10% or less, still more preferably 9% or less, still more preferably 8% or less, still more preferably 7% or less, still more preferably 6% or less, still more preferably 5% or less, still more preferably 4% or less, still more preferably 3% or less, still more preferably 2% or less, still more preferably 1% or less, or still more preferably equal to or lower than the detection limit.

An "endodermal cell marker" refers to a gene specific to endodermal cells. Examples thereof may include SOX17, FOXA2, CXCR4, AFP, GATA4, and EOMES. Endodermal cells differentiate into the digestive tract, tissues of organs such as the lung, thyroid, pancreas, and liver, cells of secretory glands opening to the digestive tract, peritoneum, pleura, larynx, auditory tube, trachea, bronchi, urinary tracts (bladder, most of the urethra, and part of the ureter), and the like.

A "mesodermal cell marker" refers to a gene specific to mesodermal cells. Examples thereof may include TBXT (BRACHYURY), MESP1, MESP2, FOXF1, HAND1, EVX1, IRX3, CDX2, TBX6, MIXL1, ISL1, SNAI2, FOXC1, and PDGFRα. Mesodermal cells differentiate into body cavities with lining mesothelium, muscle, skeleton, skin dermis, connective tissue, heart, blood vessels (including vascular endothelium), blood (including blood cells), lymph vessels, spleen, kidneys, ureters, gonads (testis, uterus, and gonadal epithelium), and the like.

An "ectodermal cell marker" refers to a gene specific to ectodermal cells. Examples thereof may include FGF5, NESTIN, SOX1, and PAX6. Ectodermal cells form the epidermis of the skin, epithelium of the terminal urethra in males, hair, nails, skin glands (including mammary and sweat glands), sensory organs (including the terminal epithelium of the oral cavity, pharynx, nose, and rectum, and salivary glands), lens, peripheral nervous system, and the like. In addition, part of the ectoderm is invaginated into grooves during development to form the neural tube, and also serves as the origin of neurons, melanocytes, and the like in the central nervous system, such as the brain and spinal cord.

The expression levels of these three germ layer markers (endodermal cell marker, mesodermal cell marker, and ectodermal cell marker) can be measured by any detection method in the art. Examples of the method for measuring the expression of three germ layer markers (endodermal cell marker, mesodermal cell marker, and ectodermal cell marker) include, but are not limited to, quantitative real-time PCR analysis, the RNA-Seq method, northern hybridization, or hybridization method using DNA array. In quantitative real-time PCR analysis, the expression level of the marker gene to be measured is converted into the relative expression level with respect to the expression level of an internal standard gene, and the expression level of the marker gene can be evaluated based on the relative expression level. Examples of the internal standard gene include the glyceraldehyde-3-phosphate dehydrogenase (GAPDH) gene and the β-actin (ACTB) gene.

On the other hand, the efficiency of differentiation of a pluripotent stem cell obtained can be determined on the basis of three germ layer markers (endodermal cell marker, mesodermal cell marker, and ectodermal cell marker) in the cell after differentiation-inducing treatment. For example, a pluripotent stem cell can be evaluated as having excellent efficiency of differentiation in a case where the expression of such a marker is enhanced in a relatively short time after the start of culture for inducing differentiation. In this case, the efficiency of differentiation may be evaluated by verifying together that the expression of the above-described pluripotent stem cell marker is decreased.

In particular, in a case where a medium not substantially containing any of a PKCβ inhibitor and a WNT inhibitor is used as the second liquid medium, all of an endodermal cell marker, mesodermal cell marker, and ectodermal cell marker can be used as an index of the efficiency of differentiation of a pluripotent stem cell. In addition, in a case where a medium containing a WNT inhibitor, and not substantially containing a PKCβ inhibitor is used as the second liquid medium, an ectodermal cell marker can be used as an index of the efficiency of differentiation of a pluripotent stem cell into in an ectodermal cell. Furthermore, in a case where a medium containing a PKCβ inhibitor and not substantially containing a WNT inhibitor is used as the second liquid medium, a mesodermal cell marker and an endodermal cell marker can be used as an index of the efficiency of differentiation of a pluripotent stem cell into a mesendodermal cell.

For example, the efficiency of differentiation of a pluripotent stem cell after suspension culture using the second liquid medium can be rated enhanced in a case where the pluripotent stem cell after suspension culture using the second liquid medium is cultured for inducing differentiation, where the culture time is measured until the positive rate of any of an endodermal cell marker, a mesodermal cell marker, and an ectodermal cell marker becomes, for example, 25% or more, 30% or more, 40% or more, 50% or more, 60% or more, 70% or more, 80% or more, 90% or more, 100%, and where the culture time measured is significantly short, compared with a case where a pluripotent stem cell after suspension culture using the first liquid medium is induced to differentiate.

In this regard, examples of a method for detecting three germ layer markers include, but are not limited to, flow cytometry. For example, in a case where a fluorescence-labeled antibody is used as a detection reagent in flow cytometry, and where a cell emitting stronger fluorescence than a negative control (isotype control or FMO control) is detected, the cell can be determined to be "positive" for the marker. Then, the ratio of cells that present positivity can be defined as a "positive rate".

In one aspect, a pluripotent stem cell collected after termination of a suspension culture step using the first liquid medium can be inoculated into the second liquid medium not substantially containing a PKCβ inhibitor and/or a WNT inhibitor to start suspension culture. In this case, washing the pluripotent stem cell collected makes it possible to avoid bringing in a PKCβ inhibitor and a WNT inhibitor contained in the first liquid medium.

In another aspect, in a case where medium change is performed continuously using a perfusion process in a suspension culture step using the first liquid medium, gradually decreasing the concentration a PKCβ inhibitor and/or a WNT inhibitor allows a transfer to a suspension culture step using the second liquid medium not substantially containing a PKCβ inhibitor and/or a WNT inhibitor. In this case, as a medium to be used for perfusion in a suspension culture step using the first liquid medium, the second liquid medium not substantially containing a PKCβ inhibitor and/or a WNT inhibitor can be used. In addition, continuing perfusion with gradually decreasing the concentration of a PKCβ inhibitor and/or a WNT inhibitor contained in a medium used for perfusion in a suspension culture step using the first liquid medium also allows a transfer to a suspension culture step using the second liquid medium not substantially containing a PKCβ inhibitor and/or a WNT inhibitor.

### (Culture Time)

In the present step of suspension culture of a pluripotent stem cell in the second liquid medium, the culture time can be set to enhance the efficiency of differentiation of a pluripotent stem cell obtained, in which the efficiency of differentiation can be defined as described above. That is, the lower limit of the culture time can be set to a culture time that allows enhancement of the efficiency of differentiation of a pluripotent stem cell obtained, in which the efficiency of differentiation can be defined as described above. This culture time varies depending on the cell line and quality of a pluripotent stem cell to be used, the medium conditions, the culture conditions, and the like, and thus, cannot be determined uniformly. By way of example, a preparatory experiment is performed under the same conditions in terms of the kind of a pluripotent stem cell to be used, the medium conditions, the culture conditions, the differentiation-inducing treatment conditions, and the like, and the culture time can be set so that the positive rate after the differentiation-inducing treatment can be significantly high. More specifically, the final stage of the suspension culture step using the second liquid medium can be set to a stage after, for example, 8 hours, 12 hours, 24 hours, 48 hours, 60 hours, 72 hours, 84 hours, 96 hours, 108 hours, 120 hours, 132 hours, or 144 hours beginning on the start of suspension culture of a pluripotent stem cell in the second liquid medium.

By the way, in the present step of suspension culture of a pluripotent stem cell in the second liquid medium, the medium not substantially containing a PKCβ inhibitor and/or a WNT inhibitor is used, and thus, the culture time, if prolonged, does not allow maintenance of the undifferentiated state, in some cases. Accordingly, the suspension culture step using the second liquid medium is preferably terminated in the range in which a pluripotent stem cell can maintain an undifferentiated state.

The culture time in the present step of suspension culture of a pluripotent stem cell in the second liquid medium varies depending on the kind of a pluripotent stem cell to be used, the medium conditions, the culture conditions, and the like, and thus, cannot be determined uniformly. By way of example, a preparatory experiment is performed under the same conditions in terms of the kind of a pluripotent stem cell to be used, the medium conditions, the culture conditions, and the like, and the culture time can be set as a condition that the rate of cells positive for any of the endodermal cell marker, mesodermal cell marker, and ectodermal cell marker is preferably 20% or less, more preferably 10% or less, still more preferably 9% or less, still more preferably 8% or less, still more preferably 7% or less, still more preferably 6% or less, still more preferably 5% or less, still more preferably 4% or less, still more preferably 3% or less, still more preferably 2% or less, still more preferably 1% or less, or still more preferably equal to or lower than the detection limit.

In addition, the culture time in the present step can be set, using, as an index, the lactic acid concentration having an adverse effect on a cell, in addition to the above-described conditions for maintaining the undifferentiatedness. In the present step, the culture time can be set so that the concentration of lactic acid accumulated in the culture solution is 15 mM or less, 14 mM or less, 13 mM or less, 12 mM or less, or 10 mM or less. The culture time is preferably set so that the concentration of lactic acid accumulated in the culture solution is particularly 9 mM or less, 8 mM or less, 7 mM or less, 6 mM or less, or 5 mM or less. As long as the concentration of lactic acid accumulated in the culture solution is within this range, an adverse effect on a cell can be avoided.

Furthermore, the culture time in the present step can be set also on the basis of the size of a cell mass in addition to the above-described conditions for maintaining the undifferentiatedness. For example, the upper limit of the size of the cell mass in the present step can be, for example, 800 µm, 700 µm, 600 µm, 500 µm, 400 µm, 300 µm, 250 µm, or 200µm. In addition, the lower limit of the size of the cell mass can be, for example, 50 µm, 100 µm, or 150µm. A cell mass (among others, a cell mass having a smaller size) within this range makes it easy for oxygen and nutrients to be supplied to the cells inside, and thus, is preferable as a growth environment for cells. At a time when a medium sampled in the suspension culture using the second liquid medium contains a cell mass of such a size, the time can be considered to be the final stage of the suspension culture step using the second liquid medium. Alternatively, the final stage of the suspension culture step using the second liquid medium can be a stage at which the cell masses defined as falling within the above-described range have come to account for 10% or more, 20% or more, 30% or more, 40% or more, 50% or more, 60% or more, 70% or more, 75% or more, 80% or more, 85% or more, 90% or more, 95% or more, 98% or more, or 100% of the cell masses observed, on a volume basis.

By way of example, the final stage of the suspension culture step using the second liquid medium can be set to a time when, for example, after 12 hours, 24 hours, 48 hours, 60 hours, 72 hours, 84 hours, 96 hours, 108 hours, 120 hours, 132 hours, or 144 hours has passed since the start of suspension culture of a pluripotent stem cell in the second liquid medium.

### 2-2-3. Collection Step

The "collection step" is a step in which cells or cell masses cultured are collected from a culture solution after a suspension culture step using the first liquid medium or a suspension culture step using the second liquid medium, and is an optional step in the method according the present invention.

As used herein, "collection (of cells)" refers to obtaining cells by separating the culture solution from the cells. A method for collecting cells may be in accordance with, but is not particularly limited to, a conventional method used in a cell culture method in the art.

Cells are present in a suspended state in the culture solution after the suspension culture step using the first liquid medium and the suspension culture step using the second liquid medium. Accordingly, the collection of cells can be achieved by removing the liquid component from the supernatant by standing or centrifugation. In addition, cells can also be collected using a filtration filter, a hollow fiber separation membrane, or the like. In the case of removing the liquid component by standing, a container containing a culture solution is allowed to stand still for approximately 5 minutes, and the supernatant may be removed, leaving the sedimented cells or cell masses. Alternatively, centrifugation may be performed using the centrifugal acceleration and the treatment time that do not cause the centrifugal force to damage the cells. For example, the lower limit of the centrifugal acceleration is not particularly limited, subject to enabling cells to be sedimented, and may be, for example, 100 × g or more, 300 × g or more, 800 × g or more, or 1000 × g or more. On the other hand, the upper limit may be any speed at which the centrifugal force never or hardly damages the cells, and may be, for example, 1600 × g or less, 1500 × g or less, or 1400 × g or less. In addition, the lower limit of the treatment time is not particularly limited, subject to being the time during which the centrifugal acceleration allows cells to be sedimented. The lower limit may be, for example, 30 seconds or more, 1 minute or more, 3 minutes or more, or 5 minutes or more. In addition, the upper limit thereof may be any time during which the centrifugal acceleration never or hardly causes the cells to be damaged. The upper limit may be, for example, 10 minutes or less, 8 minutes or less, 6 minutes or less, or 30 seconds or less. In the case of removing the liquid component by filtration, for example, the culture solution may be passed through a nonwoven fabric or a mesh filter for removing the filtrate, whereby the remaining cell aggregates are collected. In addition, in the case of removing the liquid component with a hollow fiber separation membrane, for example, the cells may be separated from the culture solution, and collected, using an apparatus equipped with a hollow fiber separation membrane, such as a cell concentration and washing system (Kaneka Corporation).

The cells and cell masses collected can be washed, if necessary. The washing method is not limited. For example, the washing method may be performed in the same manner as in the washing method described in "Post-step Treatment" in the above-described maintenance culture step. A buffer (such as a PBS buffer), physiological saline, or a medium (preferably basal medium) may be used as a washing liquid.

### 3. More Detailed Explanation of Method for Inducing Differentiation

### 3-1. Outline

A method for inducing differentiation of a pluripotent stem cell according to the present invention will be described below. A method for inducing differentiation according to the present invention is characterized in that a pluripotent stem cell produced by the above-described method for producing a pluripotent stem cell is used. Conventionally, in a pluripotent stem cell obtained by suspension culture in a liquid medium containing a PKCβ inhibitor and a WNT inhibitor, a differentiation-suppressing signal remains in the cell, thus necessitating recovery culture for removing the differentiation-suppressing signal. In contrast to this, a method for inducing differentiation of a pluripotent stem cell according to the present invention has excellent efficiency of differentiation of a pluripotent stem cell to be used, and thus, does not need recovery culture. A pluripotent stem cell obtained or a pluripotent stem cell stored for a predetermined period of time can be directly used for culture for inducing differentiation.

### 3-2. Method

A method for inducing differentiation of a pluripotent stem cell according to the present invention includes a step (differentiation-inducing step) of culturing, in a medium for inducing differentiation, a pluripotent stem cell obtained by the above-described method for producing a pluripotent stem cell, i.e., a method for producing a pluripotent stem cell, the method including a step of suspension culture using the first liquid medium and a subsequent step of suspension culture using the second liquid medium.

The method in the present aspect includes the above-described differentiation-inducing step as an essential step, and in addition, a collection step of collecting a pluripotent stem cell obtained by the above-described production method and a collection step of collecting a cell or a cell mass after inducing differentiation as optional steps.

### 3-2-1. Differentiation-inducing Step

Induction of differentiation means a phenomenon in which a pluripotent stem cell produced as described above is induced to differentiate into a different cell by a stimulus such as contact with another cell or a differentiation-inducing factor.

Here, the differentiation-inducing factor means a substance to be used for the purpose of promoting induction of differentiation. Examples include, but are not particularly limited to, a gene product in a living body, such as a growth factor; a low-molecular-weight compound that controls the action of a gene product in a living body; a hormone; and a bioactive substance, such as a vitamin. Specific examples of the differentiation-inducing factor include, but are not limited to, an Activin/TGFβ signal activator or inhibitor, BMP signal activator, BMP signal inhibitor, retinoic acid signal activator, hedgehog signal activator, hedgehog signal inhibitor, Notch signal inhibitor, WNT/β-catenin signal activator, JNK signal inhibitor, Src signal inhibitor, AMPK signal inhibitor, EGF signal activator, EGF signal inhibitor, HGF signal activator, and VEGF signal activator.

In the present step, the above-described pluripotent stem cell obtained by suspension culture using the second liquid medium is cultured in a medium containing a differentiation-inducing factor. A medium to which a differentiation-inducing factor is added is not particularly limited, and a medium conventionally used for inducing differentiation can be suitably used. As a medium to be used in the present step, a medium described in relation to the above-described first liquid medium and second liquid medium can be used.

In particular, a pluripotent stem cell obtained using, as the second liquid medium in the above-described production method, a medium not substantially containing any of a PKCβ inhibitor and a WNT inhibitor has excellent efficiency of differentiation into an endodermal cell, mesodermal cell, or ectodermal cell, or mesendodermal cell (early differentiated cell common between an endoderm and a mesoderm). Accordingly, in this case, using the pluripotent stem cell makes it possible to efficiently obtain an endodermal cell, mesodermal cell, or ectodermal cell, or mesendodermal cell.

In addition, a pluripotent stem cell obtained using, as the second liquid medium in the above-described production method, a medium containing a WNT inhibitor and not substantially containing a PKCβ inhibitor has excellent efficiency of differentiation into an ectodermal cell. Accordingly, in this case, using the pluripotent stem cell makes it possible to efficiently obtain an ectodermal cell.

Furthermore, a pluripotent stem cell obtained using, as the second liquid medium in the above-described production method, a medium containing a PKCβ inhibitor and not substantially containing a WNT inhibitor has excellent efficiency of differentiation into a mesendodermal cell, mesodermal cell, and endodermal cell. Accordingly, in this case, using the pluripotent stem cell makes it possible to efficiently obtain an endodermal cell, mesodermal cell, or mesendodermal cell.

### Examples

A method for producing a pluripotent stem cell will be described below in more detail with reference to Examples, but the technical scope of the present invention is not limited to the following Examples.

### (Reference Example 1: Adherent Culture of Human iPS Cell Line Ff-I14s04)

Human iPS cells of the Ff-I14s04 cell line, in a frozen state, were thawed, then seeded at 3000 cells/cm² in two 150 cm² dishes coated with iMatrix-511 (Nippi, Inc.) at 0.5 µg/cm², and underwent adherent culture at 37°C in a 5% CO₂ atmosphere. StemFit (registered trademark) AK02N (Ajinomoto Co., Inc.) was used as a culture medium. Given that the day on which the cells were seeded was day 0 of culture, the whole amount of the culture medium was changed on day 1 of culture, day 4 of culture, and day 6 of culture. The amount of the medium was 60 mL/dish for medium change only on day 1 of culture, and 30 mL/dish on the other days. Y-27632 (Fujifilm Wako Pure Chemical Corporation) was added to the medium, thereby yielding a final concentration of 10 µM only during cell seeding. On day 7 of culture, the cells were treated for a passage with TrypLE^{™} Select Enzyme (Thermo Fisher Scientific Inc.) supplemented with 10 µM of Y-27632 (Fujifilm Wako Pure Chemical Corporation), and the cells were detached from the culture surface, simultaneously being dispersed in single-cell form. The cells were suspended in StemFit (registered trademark) AK02N (Ajinomoto Co., Inc.) containing Y-27632 at a final concentration of 10 µM and IWR-1-endo (Fujifilm Wako Pure Chemical Corporation) at a final concentration of 20 µM, and collected.

### (Production Example 1: Suspension Culture of Human iPS Cell Line Ff-I14s04)

Suspension culture was performed using the cells collected in Reference Example 1. A single-use bottle (Satake MultiMix Corporation) was used as a culture container, and in addition, a VMF reactor (Satake MultiMix Corporation) was used as a reactor system for controlling culture. The DO sensor, a pH sensor provided with the reactor, was calibrated according to the method specified by the manufacturer. The cells were seeded such that the culture solution amount was 300 mL and the cell density at the start of culture was 1 × 10⁵ cells/mL, and then the culture was started. StemFit (registered trademark) AK02N (Ajinomoto Co., Inc.) supplemented with Y-27632 at a final concentration of 10 µM and IWR-1-endo at a final concentration of 20 µM was used as a medium for seeding. During the culture, the culture temperature was maintained at 37°C, the amount of gas supplied was maintained at 0.1 L/min, and carbon dioxide gas supply was automatically controlled so that the pH could be maintained at 7.1 to 7.2. The gas supplied was prepared by mixing air with an arbitrary volume of carbon dioxide gas, and allowed to pass on the top surface of the culture solution. The stirring rate was 80 mm/sec. Culture was started on day 0 of culture, and the whole amount of the medium was changed on day 1 of culture and day 2 of culture. For culture medium change on day 1 of culture, StemFit (registered trademark) AK02N (Ajinomoto Co., Inc.) supplemented with Y-27632 at a final concentration of 6.9 µM, IWR-1-endo at a final concentration of 20 µM, and LY-333531 at a final concentration of 1 µM was used. For culture medium change of day 2 of culture, StemFit (registered trademark) AK02N (Ajinomoto Co., Inc.) supplemented with Y-27632 at a final concentration of 3.0 µM, IWR-1-endo at a final concentration of 20 µM, and LY-333531 at a final concentration of 1 µM was used. On day 3 of culture, cell aggregates were collected, treated with TrypLE SELECT (Thermo Fisher Scientific Inc.), and dissociated by pipetting.

### (Production Example 2: Suspension Culture of Human iPS Cell Line Ff-I14s04)

The cells collected and unicellularized in Production Example 1 underwent suspension culture, and were collected in the same manner as in Production Example 1 except that StemFit (registered trademark) AK02N (Ajinomoto Co., Inc.) supplemented with Y-27632 at a final concentration of 10 µM, IWR-1-endo at a final concentration of 20 µM, and LY-333531 at a final concentration of 1 µM was used as a medium for seeding.

### (Comparative Example 1: Suspension Culture of Human iPS Cell Line Ff-I14s04, Using PKCβ Inhibitor and WNT Inhibitor)

Suspension culture was performed using the cells collected in Production Example 2. Using a 30 mL reactor (ABLE Corporation) as a culture container, the cells were cultured at a stirring rate of 100 rpm in an incubator under conditions at 37°C in 5% CO₂. The cells were seeded such that the amount of the culture solution was 30 mL, and that the cell density at the start of culture was 1 × 10⁵ cells/mL. Then, the culture was started. StemFit (registered trademark) AK02N (Ajinomoto Co., Inc.) supplemented with Y-27632 at a final concentration of 10 µM, IWR-1-endo at a final concentration of 20 µM, and LY-333531 at a final concentration of 1 µM was used as a medium for seeding. Culture was started on day 0 of culture, and the whole amount of the medium was changed on day 1 of culture and day 2 of culture. For culture medium change on day 1 of culture, StemFit (registered trademark) AK02N (Ajinomoto Co., Inc.) supplemented with Y-27632 at a final concentration of 7.7 µM, IWR-1-endo at a final concentration of 20 µM, and LY-333531 at a final concentration of 1 µM was used. For culture medium change of day 2 of culture, StemFit (registered trademark) AK02N (Ajinomoto Co., Inc.) supplemented with Y-27632 at a final concentration of 3.6 µM, IWR-1-endo at a final concentration of 20 µM, and LY-333531 at a final concentration of 1 µM was used. On day 3 of culture, cell aggregates were collected, treated with TrypLE SELECT (Thermo Fisher Scientific Inc.), and unicellularized by pipetting.

### (Comparative Example 2: Suspension Culture of Human iPS Cell Line Ff-I14s04, Using PKCβ Inhibitor and WNT Inhibitor at Concentrations Decreased Gradually)

Culture was performed in the same manner as in Comparative Example 1 except that, when suspension culture was performed using the cells collected in Production Example 2, the medium used for medium change on day 1 of culture was StemFit (registered trademark) AK02N (Ajinomoto Co., Inc.) supplemented with Y-27632 at a final concentration of 7.7 µM, IWR-1-endo at a final concentration of 11.8 µM, and LY-333531 at a final concentration of 0.59 µM, and the medium used for medium change on day 2 of culture was StemFit (registered trademark) AK02N (Ajinomoto Co., Inc.) supplemented with Y-27632 at a final concentration of 3.6 µM, IWR-1-endo at a final concentration of 2.9 µM, and LY-333531 at a final concentration of 0.15 µM.

### (Example 1: Suspension Culture of Human iPS Cell Line Ff-I14s04)

Culture was performed in the same manner as in Comparative Example 1 except that, when suspension culture was performed using the cells collected in Production Example 2, the medium used for medium change on day 1 of culture was StemFit (registered trademark) AK02N (Ajinomoto Co., Inc.) supplemented with Y-27632 at a final concentration of 7.7 µM, and the medium used for medium change on day 2 of culture was StemFit (registered trademark) AK02N (Ajinomoto Co., Inc.) supplemented with Y-27632 at a final concentration of 3.6 µM.

### (Example 2: Suspension Culture of Human iPS Cell Line Ff-I14s04, Using PKCβ Inhibitor)

Culture was performed in the same manner as in Comparative Example 1 except that, when suspension culture was performed using the cells collected in Production Example 2, the medium used for medium change on day 1 of culture was StemFit (registered trademark) AK02N (Ajinomoto Co., Inc.) supplemented with Y-27632 at a final concentration of 7.7 µM and LY-333531 at a final concentration of 1 µM, and the medium used for medium change on day 2 of culture was StemFit (registered trademark) AK02N (Ajinomoto Co., Inc.) supplemented with Y-27632 at a final concentration of 3.6 µM and LY-333531 at a final concentration of 1 µM.

### (Example 3: Suspension Culture of Human iPS Cell Line Ff-I14s04, Using WNT Inhibitor)

Culture was performed in the same manner as in Comparative Example 1 except that, when suspension culture was performed using the cells collected in Production Example 2, the medium used for medium change on day 1 of culture was StemFit (registered trademark) AK02N (Ajinomoto Co., Inc.) supplemented with Y-27632 at a final concentration of 7.7 µM and IWR-1-endo at a final concentration of 20 µM, and the medium used for medium change on day 2 of culture was StemFit (registered trademark) AK02N (Ajinomoto Co., Inc.) supplemented with Y-27632 at a final concentration of 3.6 µM and IWR-1-endo at a final concentration of 20 µM.

### (Comparative Example 3: Induction of Differentiation into Mesendoderm from Human iPS Cell Cultured in Suspension Culture Using PKCβ Inhibitor and WNT Inhibitor)

The cells collected in Comparative Example 1 were seeded for suspension culture for inducing differentiation into mesendoderms. Using a 6-well plate (Sumitomo Bakelite Co., Ltd.) as a culture container, the cells were cultured at a rotation speed of 90 rpm in an incubator under conditions at 37°C in 5% CO₂. The cells were seeded such that the amount of the culture solution was 4 mL/well, and that the cell density at the start of culture was 1 × 10⁵ cells/mL, and then the culture was started. StemFit (registered trademark) AK02N (Ajinomoto Co., Inc.) (only Liquid A and Liquid B) supplemented with Y-27632 at a final concentration of 10 µM and BMP4 at a final concentration of 2 ng/mL was used as a medium for seeding. Culture was started on day 0 of culture. On day 1 of culture, the medium was changed to StemFit (registered trademark) AK02N (Ajinomoto Co., Inc.) (only Liquid A and Liquid B) supplemented with Activin A at a final concentration of 12 ng/mL, BMP4 at a final concentration of 10 ng/mL, and bFGF at a final concentration of 10 ng/mL.

### (Comparative Example 4: Induction of Differentiation into Mesendoderm from Human iPS Cell Cultured in Suspension Culture Using PKCβ Inhibitor and WNT Inhibitor Decreased Gradually)

The cells collected in Comparative Example 2 were cultured in the same manner as in Comparative Example 3, and induced to differentiate into mesendoderms.

### (Example 4: Induction of Differentiation into Mesendoderm from Human iPS Cell Cultured in Suspension Culture)

The cells collected in Example 1 were cultured in the same manner as in Comparative Example 3, and induced to differentiate into mesendoderms.

### (Example 5: Induction of Differentiation into Mesendoderm from Human iPS Cell Cultured in Suspension Culture Using PKCβ Inhibitor)

The cells collected in Example 2 were cultured in the same manner as in Comparative Example 3, and induced to differentiate into mesendoderms.

### (Example 6: Induction of Differentiation into Mesendoderm from Human iPS Cell Cultured in Suspension Culture Using WNT Inhibitor)

The cells collected in Example 3 were cultured in the same manner as in Comparative Example 3, and induced to differentiate into mesendoderms.

### (Evaluation Example 1: Quantitative Real-Time PCR Analysis)

The cells collected after the cultures in Comparative Examples 1 and 2 and Examples 1, 2, and 3 were dissolved using TRIzol^{™} Reagent (Thermo Fisher Scientific Inc.). Total RNA was isolated and purified from the solution of the cells dissolved with TRIzol^{™} Reagent, using PureLink (registered trademark) RNA Mini Kit (Thermo Fisher Scientific Inc.). The concentration of the RNA purified was measured using BioSpec-nano (Shimadzu Corporation), and 500 ng of the RNA purified was fractionated. To the RNA fractionated, 2 µL of ReverTra Ace (registered trademark) qPCR RT Master mix (Toyobo Co., Ltd.) and Rnase Free dH₂O were added to prepare 10 µL of a solution, and cDNA synthesis was performed using SimpliAmp Thermal Cycler (Thermo Fisher Scientific Inc.). Reaction conditions for the cDNA synthesis were as follows: a reaction at 37°C was performed for 15 minutes, and then a reaction at 50°C for 5 minutes and a reaction at 98°C for 5 minutes were performed sequentially, followed by cooling to 4°C. The cDNA solution synthesized was diluted 100-fold with 10 mM Tris-HCl (pH 8.0; Nacalai Tesque, Inc.), and added at 5 µL/well to a 384-well PCR plate (Thermo Fisher Scientific Inc.). KOD SYBR (registered trademark) qPCR Mix (Toyobo Co., Ltd.), a forward primer prepared at 50 µM, a reverse primer prepared at 50 µM, and DEPC-treated water (Nacalai Tesque, Inc.) were mixed at a ratio of 100:1:1:48. The resulting solution mixture was added at 15 µL/well to the 384-well PCR plate, and mixed. The 384-well PCR plate was centrifuged to remove air bubbles from the wells, and quantitative real-time PCR analysis was performed using QuantStudio 7 Flex Real-Time PCR System (Thermo Fisher Scientific Inc.). Table 1 lists the reaction conditions.

**[Table 1]**

| | Step | Temp. | Time | Number of Cycle |
|---|---|---|---|---|
| 1 | Initial denaturation | 98°C | 1 min. | - |
| 2 | Denaturation | 98°C | 15 sec. | 5 cycles |
| 3 | Annealing, Elongation | 68°C | 30 sec. | |
| 4 | Denaturation | 98°C | 15 sec. | 40 cycles |
| 5 | Annealing | 60°C | 10 sec. | |
| 6 | Elongation | 68°C | 30 sec. | |
| 7 | Melting curve | 95°C | 15 sec. | - |
| | | 60°C | 1 min. | |
| | | 98°C | 15 sec. | |

The base sequences of the primers used for quantitative real-time PCR analysis are shown below.
ACTB (Forward): 5'-CCTCATGAAGATCCTCACCGA-3' (SEQ ID NO: 1)
ACTB (Reverse): 5'-TTGCCAATGGTGATGACCTGG-3' (SEQ ID NO: 2)
OCT4 (Forward): 5'-AGTGGGTGGAGGAAGCTGACAAC-3' (SEQ ID NO: 3)
OCT4 (Reverse): 5'-TCGTTGTGCATAGTCGCTGCTTGA-3' (SEQ ID NO: 4)
SOX2 (Forward): 5'-CACCAATCCCATCCACACTCAC-3' (SEQ ID NO: 5)
SOX2 (Reverse): 5'-GCAAAGCTCCTACCGTACCAC-3' (SEQ ID NO: 6)
NANOG (Forward): 5'-AGCCTCCAGCAGATGCAAGAACTC-3' (SEQ ID NO: 7)
NANOG (Reverse): 5'-TTGCTCCACATTGGAAGGTTCCCA-3' (SEQ ID NO: 8)
TBXT (Forward): 5'-TCACAAAGAGATGATGGAGGAAC-3' (SEQ ID NO: 9)
TBXT (Reverse): 5'-ACATGCAGGTGAGTTGTCAG-3' (SEQ ID NO: 10)
SOX17 (Forward): 5'-ATCTGCACTTCGTGTGCAAG-3' (SEQ ID NO: 11)
SOX17 (Reverse): 5'-GAGTCTGAGGATTTCCTTAGCTC-3' (SEQ ID NO: 12)
PAX6 (Forward): 5'-AGGAATGGACTTGAAACAAGG-3' (SEQ ID NO: 13)
PAX6 (Reverse): 5'-GCAAAGCTTGTTGATCATGG-3' (SEQ ID NO: 14)

The results obtained by measuring the gene expression using quantitative real-time PCR analysis are shown in Figure 1. It has been revealed that, in each specimen, the expression of each of the undifferentiation markers (OCT4, NANOG, and SOX2) was sufficiently high, and the expression of each of the differentiation markers (TBXT (mesoderm marker gene), SOX17 (endoderm marker gene), and PAX6 (ectoderm marker gene)) was suppressed and sufficiently low. In addition, in suspension culture immediately before suspension culture for inducing differentiation, the conditions under which a medium not containing one or any of a PKCβ inhibitor and a WNT inhibitor was used all yielded the same results, compared with the condition under which a medium containing both of the inhibitors was used. This has revealed that, , if suspension culture immediately before suspension culture for inducing differentiation is used, a pluripotent stem cell maintaining an undifferentiated state at a high degree can be suspension-cultured, even when a medium not containing one or any of a PKCβ inhibitor and a WNT inhibitor is used.

### (Evaluation Example 2: Quantitative Real-Time PCR Analysis)

In Evaluation Example 2, the undifferentiation markers and the differentiation markers for the cells after inducing differentiation into mesoderms were measured by quantitative real-time PCR analysis. That is, using the cells on day 2 of culture in Comparative Examples 3 and 4 and Examples 4, 5, and 6, quantitative real-time PCR analysis was performed in the same manner as in Evaluation Example 1. In Evaluation Example 2, NANOG as an undifferentiation marker and TBX3 and MESP1 as mesoderm differentiation markers were measured.

The base sequences of the primers used for quantitative real-time PCR analysis are shown below.
ACTB (Forward): 5'-CCTCATGAAGATCCTCACCGA-3' (SEQ ID NO: 1)
ACTB (Reverse): 5'-TTGCCAATGGTGATGACCTGG-3' (SEQ ID NO: 2)
NANOG (Forward): 5'-AGCCTCCAGCAGATGCAAGAACTC-3' (SEQ ID NO: 7)
NANOG (Reverse): 5'-TTGCTCCACATTGGAAGGTTCCCA-3' (SEQ ID NO: 8)
TBX3 (Forward): 5'-TACCAAGTCGGGAAGGCGAA-3' (SEQ ID NO: 15)
TBX3 (Reverse): 5'-GGGCTGTCCGGGTGAATGTA-3' (SEQ ID NO: 16)
MESP1 (Forward): 5'-AGCCCAAGTGACAAGGGACAACT-3' (SEQ ID NO: 17)
MESP1 (Reverse): 5'-AAGGAACCACTTCGAAGGTGCTGA-3' (SEQ ID NO: 18)

The results obtained by measuring the gene expression using quantitative real-time PCR analysis are shown in Figure 2. It has been revealed that, in Examples 4 and 5 in which differentiation was induced using the pluripotent stem cells obtained immediately after the suspension culture in a medium not containing a WNT inhibitor, the expression of each of the differentiation markers TBX3 and MESP1 was noticeably high, and the expression of the undifferentiation marker NANOG was noticeably decreased, compared with Comparative Examples 3 and 4 and Example 6 in which differentiation was induced using the pluripotent stem cells obtained immediately after the suspension culture in a medium containing a WNT inhibitor. This result has revealed that using a medium not containing a WNT inhibitor in suspension culture immediately before suspension culture for inducing differentiation allowed induction of differentiation into a mesendoderm to progress more efficiently. In other words, it has been revealed that in a case where a pluripotent stem cell was suspension-cultured using a WNT inhibitor to suppress a decrease in quality due to spontaneous differentiation into a mesendoderm, a strong differentiation-suppressing signal remained in the cells used for inducing differentiation immediately after the suspension culture, and thus, using the cells resulted in inhibiting differentiation into a mesendoderm of interest.

In other words, it has been revealed that in a case where a pluripotent stem cell was suspension-cultured using a PKCβ inhibitor to suppress a decrease in quality due to spontaneous differentiation into an ectoderm, a strong differentiation-suppressing signal remained in the cells used for inducing differentiation immediately after the suspension culture, and thus, using the cells resulted in inhibiting differentiation into an ectoderm of interest. However, it can be said that it is highly probable that using a medium not containing a PKCβ inhibitor in suspension culture immediately before suspension culture for inducing differentiation allows induction of differentiation into an ectoderm to progress more efficiently.

Sequencing Listing

## Claims

1. A method for producing a pluripotent stem cell, comprising:
a step of suspension-culturing the pluripotent stem cell in a first liquid medium containing a PKCβ inhibitor and a WNT inhibitor; and
a subsequent step of suspension-culturing the pluripotent stem cell in a second liquid medium not substantially containing a PKCβ inhibitor and/or a WNT inhibitor.

2. The production method according to claim 1, wherein the second liquid medium does not substantially contain any one of the PKCβ inhibitor and the WNT inhibitor, and contains the other.

3. The production method according to claim 1, comprising a step of changing the first liquid medium to the second liquid medium.

4. The production method according to claim 1, wherein the concentration of the WNT inhibitor contained in the second liquid medium is less than 90 nM.

5. The production method according to claim 1, wherein the concentration of the PKCβ inhibitor contained in the second liquid medium is less than 25 nM.

6. The production method according to claim 1, wherein the first liquid medium contains the WNT inhibitor with the concentration of 90 nM or more and 40 µM or less and the PKCβ inhibitor with the concentration of25 nM or more and 15 µM or less.

7. The production method according to claim 1, wherein a cell population in which the proportion of cells positive for OCT4 is 90% or more, the proportion of cells positive for SOX2 is 90% or more, and the proportion of cells positive for NANOG is 90% or more is produced by the step of suspension-culturing the pluripotent stem cell in the second liquid medium.

8. The production method according to claim 1, wherein the second liquid medium is a medium containing a PKCβ inhibitor and not substantially containing a WNT inhibitor, and wherein the pluripotent stem cell suitable for differentiation into a mesendodermal cell is produced by the step of suspension-culturing the pluripotent stem cell in the second liquid medium.

9. The production method according to claim 1, wherein the second liquid medium is a medium containing a WNT inhibitor and not substantially containing a PKCβ inhibitor, and wherein the pluripotent stem cell suitable for differentiation into an ectodermal cell is produced by the step of suspension-culturing the pluripotent stem cell in the second liquid medium.

10. A method for inducing differentiation of a pluripotent stem cell, comprising
a step of culturing, in a medium for inducing differentiation, the pluripotent stem cell produced by the production method according to any one of claims 1 to 9.
